# FASCICULE DE BREVET EUROPEEN

(11) **EP 0 586 490 B1**
(45) Date de publication et mention de la délivrance du brevet: **20.03.1996**
(21) Numéro de dépôt: 92911474.2
(22) Date de dépôt: 15.05.1992
(51) Int. Cl.: C07D 209/44, A61K 31/40

(54) **DERIVES DE PERHYDROISOINDOLE, LEUR PREPARATION ET LES COMPOSITIONS PHARMACEUTIQUES QUI LES CONTIENNENT**
PERHYDROISOINDOLDERIVATE, IHRE HERSTELLUNG UND DIESE ENTHALTENDE ARZNEIMITTEL
DERIVATIVES OF PERHYDROISOINDOL, THEIR PREPARATION, AND PHARMACEUTICAL COMPOSITIONS CONTAINING THEM

(30) Priorité: 17.05.1991 FR 9106035
(43) Date de publication de la demande: 16.03.1994
(73) Titulaire: RHONE-POULENC RORER S.A., F-92160 Antony (FR)
(72) Inventeur: ACHARD, Daniel, F-94320 Thiais (FR); GRISONI, Serge, F-94600 Choisy-le-Roi (FR); HANESSIAN, Stephen, Beaconsfields, Quebec H9W 5H3 (CA); MOUTONNIER, Claude, F-92350 Le Plessis-Robinson (FR); PEYRONEL, Jean-François, F-91120 Palaiseau (FR); TABART, Michel, F-75013 Paris (FR); TRUCHON, Alain, F-69004 Lyon (FR)
(74) Mandataire: Savina, Jacques
(86) Numéro de dépôt international: FR9200429
(87) Numéro de publication internationale: WO9220653

(56) Documents cités:
- EP-A- 0 429 366
- EP-A- 0 430 771
- WO-A-90/05525

## Description

La présente invention concerne de nouveaux dérivés du perhydroisoindole de formule générale : 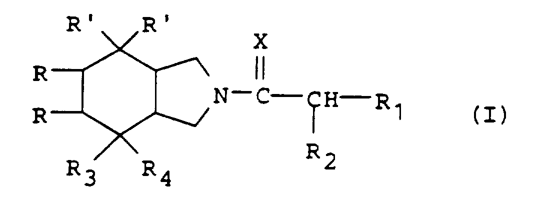 ainsi que leurs sels lorsqu'ils existent, qui antagonisent les effets de la substance P et sont de ce fait particulièrement intéressants dans les domaines thérapeutiques où cette substance est connue pour intervenir.

Dans le brevet américain 4 042 707 avaient été décrits des produits dérivés de l'isoindole de formule générale: 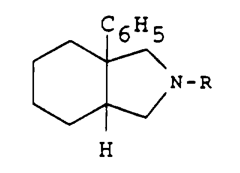 ayant une activité opiacée.

Ces produits n'ont pas d'activité vis à vis de la substance P.

Jusqu'à présent, malgré les recherches mises en oeuvre et malgré l'intérêt suscité [M.R. Hanley, TINS, (5) 139 (1982)], il n'avait pratiquement pas été découvert de produit agissant spécifiquement sur la substance P et ayant une structure non peptidique, c'est pourquoi les dérivés de l'isoindole de formule générale (I) présentent un intérêt considérable.

Dans la formule générale (I) :
- les radicaux R sont identiques et représentent des atomes d'hydrogène ou forment ensemble une liaison,
- les symboles R' sont identiques et représentent des radicaux phényle éventuellement substitués par un atome d'halogène ou par un radical méthyle en position 2 ou 3,
- le symbole X représente un atome d'oxygène, ou un radical NH,
- le symbole R₁ représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino) alcoyloxy ou alcoylthio pouvant être éventuellement substitués [par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy, hydroxyalcoyle)], ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle ou hétérocyclyle mono ou polycyclique, saturé ou insaturé contenant 5 à 9 atomes de carbone et un ou plusieurs hétéroatomes choisis parmi l'oxygène, l'azote ou le soufre,
- le symbole R₂ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino, acylamino ou alcoyloxycarbonylamino,
- le symbole R₃ représente un atome d'halogène ou un radical hydroxy et
- le symbole R₄ représente un atome d'hydrogène ou simultanément à R₃ représente un atome d'halogène.

Il est entendu que les radicaux alcoyle ou acyle cités ci-dessus contiennent 1 à 4 atomes de carbone en chaîne droite ou ramifiée.

Lorsque R' porte un substituant halogène, ce dernier peut être choisi parmi le chlore ou le fluor.

Lorsque R₁ contient un atome d'halogène, ce dernier peut être choisi parmi le chlore, le brome, le fluor ou l'iode.

Lorsque R₁ représente un radical hétérocyclyle mono ou polycyclique, saturé ou insaturé, à titre d'exemple il peut être choisi parmi thiényle, furyle, pyridyle, dithiinyle, indolyle, isoindolyle, thiazolyle, isothiazolyle, oxazolyle, imidazolyle, pyrrolyle, triazolyle, thiadiazolyle, quinolyle, isoquinolyle, ou naphtyridinyle.

Lorsque R₁ représente phényle substitué par une chaîne portant un hétérocycle, ce dernier peut être choisi parmi pyrrolidinyle, morpholino, pipéridinyle, tétrahydropyridinyle pipérazinyle, thiomorpholino.

Lorsque R₃ est un atome d'halogène , il peut être avantageusement choisi parmi le fluor ou le chlore.

Par ailleurs, les produits de formule générale (I) présentent différentes formes stéréoisomères, il est entendu que les dérivés de l'isoindole de forme (3aR,7aR) à l'état pur, ou sous forme de mélange des formes cis (3aRS,7aRS), entrent dans le cadre de la présente invention. Lorsque les radicaux R₃ et R₄ sont différents, il est également entendu que le substituant R₃ peut être en position axiale ou équatoriale et de ce fait que les dérivés R et S ainsi que leurs mélanges entrent aussi dans le cadre de le présente invention. De plus, lorsque le symbole R₂ est autre que l'atome d'hydrogène, la chaîne substituée sur l'isoindole présente un centre chiral, il est entendu que les formes stéréoisomères et leurs mélanges entrent aussi dans le cadre de la présente invention.

Selon l'invention les dérivés du perhydroisoindole de formule générale (I) peuvent être obtenus par action de l'acide de formule générale : 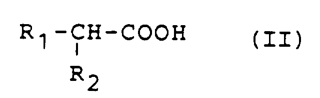 ou d'un dérivé réactif de cet acide, dans lequel R₁ et R₂sont définis comme précédemment, sur un dérivé de l'isoindole de formule générale : 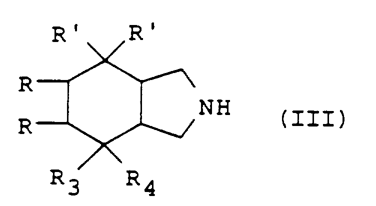 dans laquelle les symboles R, R', R₃ et R₄ sont définis comme précédemment suivie le cas échéant de la transformation de l'amide obtenu en une amidine .

Il est entendu que, les radicaux amino, alcoylamino ou carboxy contenus dans R₁ et/ou R₂ sont de préférence préalablement protégés. La protection s'effectue par tout groupement compatible, dont la mise en place et l'élimination n'affectent pas le reste de la molécule. Notamment, on opère selon les méthodes décrites par T.W. Greene, Protective Groups in Organic Synthesis, A. Wiley -Interscience Publication (1981), ou par Mc Omie, Protective Groups in Organic Chemistry, Plenum Press (1973).

A titre d'exemple,
- les groupements amino ou alcoylamino peuvent être protégés par des radicaux méthoxycarbonyle, éthoxycarbonyle, t.butoxycarbonyle, allyloxycarbonyle, vinyloxycarbonyle, trichloréthoxycarbonyle, trichloracétyle, trifluoracétyle, chloracétyle, trityle, benzhydryle, benzyle, allyle, formyle, acétyle, benzyloxycarbonyle ou ses dérivés substitués;
- les groupements acides peuvent être protégés par des radicaux méthyle, éthyle, t.butyle, benzyle, benzyle substitué ou benzhydryle.

De plus, lorsque R₂ représente un radical hydroxy, il est préférable de protéger préalablement ce radical. La protection s'effectue par exemple par un radical acétyle, trialcoylsilyle, benzyle, ou sous forme d'un carbonate par un radical -COORa dans lequel Ra est un radical alcoyle ou benzyle.

Lorsque l'on effectue la condensation d'un dérivé réactif de l'acide de formule générale (II), on opère avantageusement au moyen du chlorure d'acide, de l'anhydride, d'un anhydride mixte ou d'un ester réactif dans lequel le reste de l'ester est un radical succinimido, benzotriazolyl-1 éventuellement substitué, nitro-4 phényle, dinitro-2,4 phényle, pentachlorophényle ou phtalimido.

La réaction s'effectue généralement à une température comprise entre -40 et +40°C, dans un solvant organique tel qu'un solvant chloré (dichlorométhane, dichloréthane, chloroforme par exemple), un hydrocarbure (toluène par exemple), un éther (tétrahydrofuranne, dioxanne par exemple), un ester (acétate d'éthyle par exemple), un amide (diméthylacétamide, diméthylformamide par exemple), ou une cétone (acétone par exemple) ou dans un mélange de ces solvants, en présence d'un accepteur d'acide tel qu'une base organique azotée comme par exemple la pyridine, la diméthylaminopyridine, la N-méthylmorpholine ou une trialcoylamine (notamment triéthylamine) ou tel qu'un époxyde (oxyde de propylène par exemple). Il est également possible d'opérer en présence d'un agent de condensation tel qu'un carbodiimide, [par exemple dicyclohexylcarbodiimide ou (diméthylamino-3 propyl)-1 éthyl-3 carbodiimide], le NN'-carbonyldiimidazole ou l'éthoxy-2 éthoxycarbonyl-1 dihydro-1,2 quinoléine ou bien en milieu hydroorganique,en présence d'un agent alcalin de condensation comme le bicarbonate de sodium, puis on transforme le cas échéant l'amide obtenu en une amidine telle que définie précédemment.

La transformation de l'amide de formule générale (I) en une amidine pour laquelle X est un radical NH s'effectue en préparant le dérivé de l'isoindolium de formule générale : 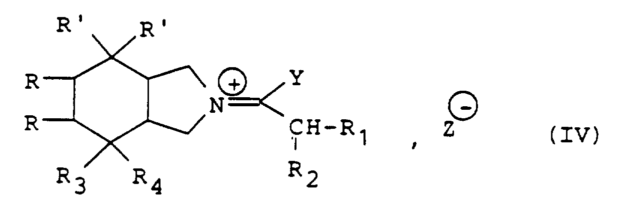 dans laquelle R, R', R₁, R_{2,} R₃ et R₄ sont définis comme précédemment, Y représente un atome de chlore, un radical méthoxy ou éthoxy et Z⁻ représente un ion chlorure, tétrafluoroborate, fluorosulfonate, trifluorométhylsulfonate, méthylsulfate, ou éthylsulfate, puis par action de l'ammoniac sur le dérivé de l'isoindolium.

Il est entendu que, lorsque R₃ est hydroxy, Y est autre qu'un atome de chlore.

La préparation du dérivé de l'isoindolium de formule générale (IV) dans laquelle Y est un atome de chlore ou un radical méthoxy ou éthoxy s'effectue par action d'un réactif tel que le phosgène, l'oxychlorure de phosphore, le pentachlorure de phosphore, le chlorure de thionyle, le chlorure d'oxalyle, le chloroformate de trichlorométhyle, le tétrafluoroborate de triéthyl (ou de triméthyl) oxonium, le triflate de méthyle (ou d'éthyle), le fluorosulfonate de méthyle (ou d'éthyle) ou le sulfate de méthyle (ou d'éthyle). La réaction s'effectue dans un solvant chloré (dichlorométhane, dichloréthane par exemple) ou dans un hydrocarbure aromatique (toluène par exemple), à une température comprise entre 0°C et la température de reflux du mélange réactionnel. L'action de l'ammoniac sur le dérivé de formule générale (IV) s'effectue dans un solvant organique anhydre tel qu'un solvant chloré (dichlorométhane, dichloréthane par exemple), dans un mélange alcool-solvant chloré, dans un éther (tétrahydrofurane par exemple), dans un ester (par exemple acétate d'éthyle), dans un solvant aromatique (toluène par exemple) ou dans un mélange de ces solvants, à une température comprise entre -20°C et la température de reflux du mélange réactionnel.

Il n'est pas indispensable d'avoir isolé le dérivé de l'isoindolium de formule générale (IV) pour le mettre en oeuvre dans cette réaction.

Selon l'invention le dérivé de l'isoindole de formule générale (I) pour lequel R₃ représente un atome d'halogène et R_{4,} représente un atome d'hydrogène peut également être obtenus par halogénation du dérivé correspondant de l'isoindole de formule générale (I) pour lequels R₃ est un radical hydroxy, R₄ est un atome d'hydrogène et X est un atome d'oxygène, puis le cas échéant transformation l'amide obtenu en une amidine.

Lorsque l'on veut obtenir un produit pour lequel R₃ représente un atome de fluor, la réaction s'effectue avantageusement au moyen d'un agent de fluoration comme un fluorure de soufre [trifluorure de morpholino soufre, tétrafluorure de soufre (J. Org. Chem., 40, 3808 (1975)), trifluorure de diéthylamino soufre (Tetrahedron, 44, 2875 (1988)), trifluorure de phényl soufre (J. Am. Chem. Soc., 84, 3058 (1962)], comme l'hexafluoropropyl diéthylamine (brevet japonnais 2 039 546) ou la. N-(chloro-2 trifluoro-1,1,2 éthyl) diéthylamine, comme le tétrafluorure de sélenium (J. Am. Chem. Soc., 96,925 (1974) ou comme le tétrafluorophénylphosphorane (Tet. Let., 907 (1973), en opèrant dans un solvant organique tel qu'un solvant chloré (dichlorométhane, dichloréthane par exemple) à une température comprise entre -30 et 30°C. Il est entendu que la mise en oeuvre de l'alcool de configuration (S) conduit au dérivé fluoré de configuration (R) et que la mise en oeuvre de l'alcool de configuration (R) conduit au dérivé fluoré de configuration (S).

Lorsque l'on veut obtenir un produit pour lequel R₃ représente un atome de chlore, le dérivé chloré de configuration (R) peut être obtenu par traitement de l'alcool (S) par le pentachlorure de phosphore dans les conditions définies par R.J. Cremlyn et coll., J. Chem. Soc., 3794 (1954) ; le dérivé chloré de configuration (S) peut être obtenu par traitement de l'alcool (S) par le chlorure de thionyle dans les conditions citées par R.J. Cremlyn dans la référence mentionnée ci-dessus.

Selon l'invention les dérivés de l'isoindole de formule générale (I) pour lesquels R₃ est un radical hydroxy et R₄ est un atome d'hydrogène, peuvent également être obtenus par réduction du dérivé de l'isoindolone de formule générale : 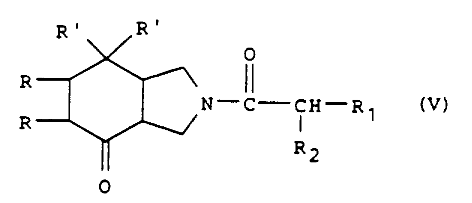 dans laquelle R, R', R₁ et R₂ sont définis comme précédemment, suivie de la séparation des isomères axial et équatorial et de la transformation de l'amide obtenu en une amidine.

La réduction s'effectue avantageusement au moyen d'un borohydrure alcalin (borohydrure de sodium, tri-s.butylborohydrure de lithium) dans un solvant tel qu'un alcool (méthanol, éthanol par exemple) ou un éther (tétrahydrofuranne) en milieu basique, à une température comprise entre -20 et 50°C.

Selon l'invention, les dérivés de l'isoindole de formule générale (I) pour lesquels X est un radical imino peuvent également être obtenus à partir du dérivé de l'isoindole de formule générale (III), par action d'un produit de formule générale : 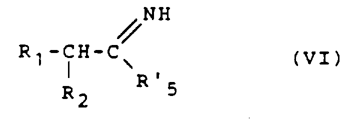 éventuellement à l'état de sel, dans laquelle R₁ et R₂ sont définis comme précédemment et R'₅ représente un radical alcoyloxy contenant 1 à 4 atomes de carbone en chaîne droite ou ramifiée ou un radical méthylthio, éthylthio, benzylthio ou alcoyloxycarbonylméthylthio.

La réaction est mise en oeuvre au moyen du dérivé de formule générale (VI) éventuellement préparé in situ, dans un solvant organique tel qu'un solvant chloré (dichlorométhane, dichloréthane par exemple), un éther (tétrahydrofuranne par exemple), un hydrocarbure aromatique (toluène par exemple) ou un nitrile par exemple l'acétonitrile à une température comprise entre 0°C et la température de reflux du mélange réactionnel.

Il est entendu que, dans l'éventualité où les radicaux R₁ et/ou R₂ du produit de formule générale (VI) portent des substituants pouvant interférer avec la réaction, ces derniers doivent être préalablement protégés.

Les acides de formule générale (II) peuvent être préparés selon les méthodes décrites ci-après dans les exemples, ou par analogie avec ces méthodes.

Le dérivé de l'isoindole de formule générale (III) pour lequel R₃ est un atome d'halogène et R₄ est un atome d'hydrogène ou d'halogène peut être préparé par halogénation d'un isoindole de formule générale : 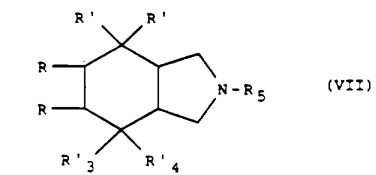 dans laquelle R et R' sont définis comme précédemment, R₅ est un radical protecteur, R'₃ est un radical hydroxy et R'₄ un atome d'hydrogène si l'on veut obtenir un dérivé de l'isoindole monohalogéné, ou bien R'₃ et R'₄ forment ensemble un radical oxo si l'on veut obtenir un dérivé de l'isoindole dihalogéné, puis élimination du radical protecteur R₅.

Le radical protecteur R₅ peut être tout groupement protecteur d'amino qui soit compatible avec la réaction et dont la mise en place et l'élimination n'altère pas le reste de la molécule. A titre d'exemple peuvent être cités les groupements alcoyloxycarbonyle, benzyloxycarbonyle, benzyle éventuellement substitués, formyle, chloracétyle, trichloacétyle, trifluoracétyle, vinyloxycarbonyle phénoxycarbonyle, chloro-1 éthoxycarbonyle ou chlorocarbonyle.

Lorsque l'on veut obtenir un dérivé fluoré de perhydroisoindole de formule générale (III), la fluoration s'effectue dans les conditions décrites précédemment pour la fluoration d'un dérivé de formule générale (I) dans laquelle R₃ est hydroxy, à une température comprise entre -30 et +30°C. Il est entendu que le dérivé fluoré de configuration (R) est obtenu à partir de l'alcool de configuration (S) et que le dérivé fluoré de configuration (S) est obtenu à partir du dérivé hydroxylé de configuration (R). Il est également possible d'opérer à partir d'un mélange des alcools de configuration (R) et (S) et d'effectuer la séparation au niveau du dérivé de formule générale (III).

Lorsque l'on veut obtenir le dérivé difluoré de formule générale (III), la réaction s'effectue à partir de l'isoindolone de formule générale (VII) (R'₃ et R'₄ forment ensemble un radical oxo), en opérant dans les conditions définies ci-dessus, à une température comprise entre 30°C et la température de reflux du mélange réactionnel.

Lorsque l'on veut obtenir un dérivé chloré du perhydroisoindole de formule générale (III) la chloration s'effectue selon les conditions décrites par R. J. Cremlyn et coll., J. Chem. Soc., 3794 (1954), soit au moyen de pentachlorure de phosphore à partir du dérivé hydroxylé de configuration (S) lorsque l'on veut obtenir le dérivé chloré de configuration (R), soit au moyen de chlorure de thionyle à partir du dérivé hydroxylé de configuration (S) lorsque l'on veut obtenir un dérivé chloré de configuration (S). Il est entendu que l'on peut aussi effectuer la séparation au niveau du produit de formule générale (III).

Lorsque l'on veut obtenir le dérivé dichloré, on opère à partir de la perhydroisoindole de formule générale (VII), par traitement par le pentachlorure de phosphore dans les conditions citées ci-dessus.

L'élimination subséquente du radical protecteur R₅ s'effectue selon les méthodes habituelles. Notamment selon les méthodes décrites par T.W. Greene, par A. Wiley ou par Mc Omie dans les références citées précédemment.

Le dérivé de l'isoindole de formule générale (III) pour lequel R₃ est un atome d'halogène et R₄ est un atome d'hydrogène, peut également être obtenu par halogénation d'un dérivé de perhydroisoindole de formule générale : 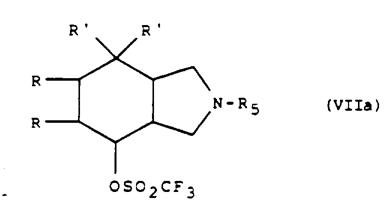 dans laquelle R et R' sont définis comme précédemment, puis élimination du radical protecteur R₅.

L'halogénation s'effectue par un halogénure d'ammonium quaternaire tel que par exemple fluorure de tétrabutylammmonium ou par un halogénure alcalin tel que le fluorure de potassium ou le fluorure de césium par exemple, en milieu anhydre, dans un solvant organique tel qu'un éther (tétrahydrofuranne, dioxanne par exemple) un solvant chloré (dichlorométhane par exemple) ou dans un mélange de solvants, à une température comprise entre -30 et 50°C.

Il est entendu que le dérivé sulfonylé de formule générale (VIIa) de configuration (S) conduit à un dérivé halogéné de configuration (R) et que le dérivé sulfonylé de configuration (R) conduit à un dérivé halogéné de configuration (S).

L'élimination du radical R₅ s'effectue comme décrit précédemment.

Le dérivé sulfonylé de formule générale (VIIa) peut être obtenu par traitement du dérivé de perhydroisoindole de formule générale (VII) pour lequel R'₃ est un radical hydroxy et R'₄ est un atome d'hydrogène, par un dérivé réactif de l'acide trifluorométhane sulfonique.

La réaction s'effectue généralement par action de l'anhydride trifluorométhane sulfonique en présence de pyridine, dans un solvant chloré (dichlorométhane par exemple), à une température voisine de - 30°C.

Le dérivé de perhydroisoindole de formule générale (VII) peut être préparé par protection de l'amino du dérivé correspondant de formule générale : 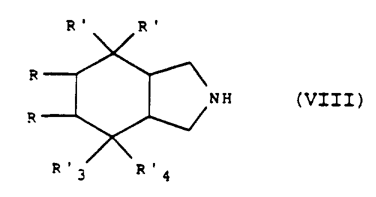 dans laquelle R, R', R'₃ et R'₄ sont définis comme pour la formule générale (VII).

La protection s'effectue selon les méthodes habituelles. Notamment selon les références citées précédemment.

Le dérivé de perhydroisoindole de formule générale (VII) ou (VIII) pour lequel R'₃ est un radical hydroxy et R'₄ est un atome d'hydrogène, peut être obtenu par réduction du dérivé correspondant du perhydroisoindole de formule générale (VII) ou (VIII) pour lequel R'₃ et R'₄ forment ensemble un radical oxo.

La réduction s'effectue dans des conditions analogues à celles décrites pour l'obtention des perhydroisoindoles de formule générale (I) pour lesquels R₃ est hydroxy, à partir de la perhydroisoindolone correspondante.

Le dérivé hydroxylé de perhydroisoindole de formule générale (III) ou (VIII) pour lequel R'₃ est un radical hydroxy et R'₄ est un atome d'hydrogène peut être obtenu par libération du radical protecteur R₅ du dérivé de perhydroisoindole correspondant de formule générale (VII) dans laquelle R'₃ et R'₄ sont définis comme ci-dessus.

L'élimination s'effectue selon les méthodes connues qui n'affectent pas le reste de la molécule.

Le dérivé de l'isoindole de formule générale (VIII) pour lequel R'₃ et R'₄ forment ensemble un radical oxo, peut être obtenu à partir du dérivé correspondant de formule générale : 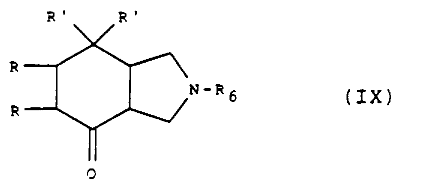 dans laquelle R et R' sont définis comme précédemment et R₆ représente un radical allyle ou un radical de structure - CRₐR_{b}R_{c} dans laquelle Rₐ et R_{b} sont des atomes d'hydrogène ou des radicaux phényle éventuellement substitués (par un atome d'halogène, un radical alcoyle, alcoyloxy ou nitro), et R_{c} est défini comme Rₐ et R_{b} ou représente un radical alcoyle ou alcoyloxyalcoyle, l'un au moins de Rₐ, R_{b} et R_{c} étant un radical phényle substitué ou non et les radicaux alcoyle contenant 1 à 4 atomes de carbone en chaîne droite ou ramifiée, par élimination du radical R₆, par toute méthode connue qui n'affecte pas le reste de la molécule.

Notamment, lorsque R est un atome d'hydrogène, et lorsque R₆ est autre qu'un radical allyle, le groupement R₆ peut être éliminé par hydrogénation catalytique en présence de palladium. Généralement, la réaction s'effectue en milieu acide, dans un solvant tel qu'un alcool (méthanol, éthanol), dans l'eau ou directement dans l'acide acétique ou l'acide formique, à une température comprise entre 20 et 60°C.

Lorsque R₆ est un radical benzhydryle ou trityle, l'élimination peut être effectuée par traitement en milieu acide, en opérant à une température comprise entre 0°C et la température de reflux du mélange réactionnel, dans un alcool, dans un éther, dans l'eau ou directement dans l'acide acétique, l'acide formique ou l'acide trifluoroacétique.

Le groupement R₆ peut être également éliminé en faisant agir le chloroformiate de vinyle, le chloroformiate de chloro-1 éthyle ou le chloroformiate de phényle, en passant intermédiairement par un produit de formule générale : 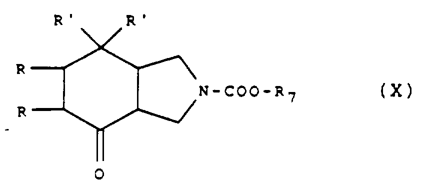 dans laquelle R et R' sont définis comme précédemment, et R₇ est un radical vinyle, chloro-1 éthyle ou phényle, puis par élimination du radical -COOR₇ par traitement acide. L'action du chloroformiate s'effectue généralement dans un solvant organique tel qu'un solvant chloré (dichlorométhane, dichloréthane, chloroforme par exemple), un éther (tétrahydrofuranne, dioxanne par exemple) ou une cétone (acétone par exemple) ou dans un mélange de ces solvants, en opérant à une température comprise entre 20°C et la température de reflux du mélange réactionnel.

L'élimination du radical -COOR₇ est effectuée par traitement en milieu acide par exemple par l'acide trifluoroacétique, formique, méthanesulfonique, p.toluènesulfonique, chlorhydrique ou bromhydrique dans un solvant tel qu'un alcool, un éther, un ester, un nitrile, un mélange de ces solvants ou dans l'eau, à une température comprise entre 0°C et la température de reflux du mélange réactionnel.

Dans les conditions d'élimination des radicaux -COOR₇ citées précédemment, le dérivé de l'isoindolone de formule générale (VIII) est obtenu à l'état de sel de l'acide employé qui peut être mis en oeuvre directement dans l'étape ultérieure.

Le dérivé de l'isoindolone de formule générale (IX) (ou (VII) lorsque R'₃ et R'₄ forment ensemble un radical oxo et R₅ est un radical benzyle éventuellement substitué), peuvent être obtenus par réaction de cycloaddition par action d'un dérivé silylé de formule générale : 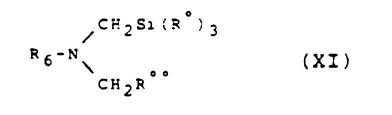 dans laquelle R₆ est défini comme précédemment, (R°)₃ représente des radicaux alcoyle ou des radicaux alcoyle et phényle et R°° représente un radical alcoyloxy, cyano ou phénylthio, sur le dérivé de la cyclohexènone de formule générale : 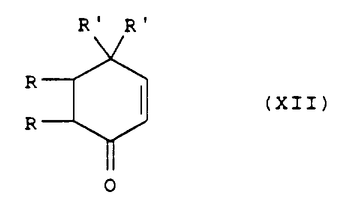 dans laquelle R et R' sont définis comme précédemment.

On opère en présence d'une quantité catalytique d'un acide choisi parmi l'acide trifluoroacétique, l'acide acétique, l'acide méthanesulfonique ou les acides cités dans les références mentionnées ci-dessous, dans un solvant organique tel qu'un solvant chloré (dichlorométhane, dichloréthane par exemple), dans un hydrocarbure aromatique, dans un nitrile (acétonitrile) ou dans un éther, à une température comprise entre 0°C et la température de reflux du mélange réactionnel.

Le dérivé silylé de formule générale (XI) peut être obtenu selon les méthodes décrites par :
- Y. Terao et coll., Chem. Pharm. Bull., 33, 2762 (1985);
- A. Hosomi et coll., Chem. Lett., 1117 (1984)
- A. Padwa et coll., Chem. Ber., 119, 813 (1986) ou
- Tetrahedron, 41, 3529 (1985).

Il est entendu que les dérivés de perhydroisoindole de formule générale (I), (III), (V), (VII), (VIIa), (VIII), (IX) et (X) présentent plusieurs formes stéréoisomères. Lorsque l'on veut obtenir un produit de formule générale (I) de forme (3aR,7aR), la séparation des formes isomères est mise en oeuvre de préférence au niveau du dérivé de formule générale (VIII) pour lequel R'₃ et R'₄ forment ensemble un radical oxo. Elle peut aussi être mise en oeuvre au niveau du dérivé de formule générale (III). La séparation s'effectue selon toute méthode connue et compatible avec la molécule.

A titre d'exemple, la séparation peut être effectuée par préparation d'un sel optiquement actif, par action de l'acide L(+) ou D(-) mandélique, ou de l'acide dibenzoyltartrique, puis séparation des isomères par cristallisation. L'isomère recherché est libéré de son sel en milieu basique.

La séparation des isomères axial et équatorial des dérivés hydroxylés ou des dérivés halogénés s'effectue avantageusement au niveau des produits de formule générale (VII) ou (VIII), en opérant par cristallisation et chromatographie. Il est également possible d'opérer au niveau des produits de formule générale (III) ou (I).

Les nouveaux dérivés de l'isoindole de formule générale (I) peuvent être purifiés le cas échéant par des méthodes physiques telles que la cristallisation ou la chromatographie.

Le cas échéant, les nouveaux dérivés de formule générale (I) pour lesquels les symboles R₁ et/ou R₂ contiennent des substituants amino ou alcoylamino et/ou X représente un radical NH, peuvent être transformés en sels d'addition avec les acides. Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, p.toluènesulfonates, iséthionates, ou avec des dérivés de substitution de ces composés).

Les nouveaux dérivés de l'isoindole de formule générale (I) peuvent aussi, le cas échéant, lorsque R₂ représente un radical carboxy, être transformés en sels métalliques ou en sels d'addition avec une base azotée, selon les méthodes connues en soi. Ces sels peuvent être obtenus par action d'une base métallique (par exemple alcaline ou alcalino terreuse), de l'ammoniac ou d'une amine, sur un produit selon l'invention, dans un solvant approprié tel qu'un alcool, un éther ou l'eau, ou par réaction d'échange avec un sel d'un acide organique. Le sel formé précipite après concentration éventuelle de la solution, il est séparé par filtration, décantation ou lyophilisation. Comme exemples de sels pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-β-phénéthylamine, NN'-dibenzyléthylènediamine, diphénylènediamine, benzhyldrylamine, quinine, choline, arginine, lysine, leucine, dibenzylamine).

Les nouveaux dérivés de l'isoindole selon la présente invention qui antagonisent les effets de la substance P peuvent trouver une application dans les domaines de l'analgésie, de l'inflammation de l'asthme, des allergies, sur le système nerveux central, sur le système cardiovasculaire, comme antispasmodique, ou sur le système immunitaire, ainsi que dans le domaine de la stimulation des secrétions lachrymales.

En effet, les produits selon l'invention manifestent une affinité pour les récepteurs à substance P à des doses comprises entre 5 et 2000 nM selon la technique décrite par C.M. Lee et coll., Mol. Pharmacol., 23, 563-69 (1983).

Il a de plus été démontré qu'il s'agit d'un effet antagoniste de la substance P, au moyen de différents produits. Dans la technique décrite par S. Rosell et coll., Substance P, Ed. by US Von Euler and B. Pernow, Raven Press, New-York (1977), pages 83 à 88, les produits étudiés se sont montrés actifs à des doses comprises entre 20 et 1000 nM.

La substance P est connue pour être impliquée dans un certain nombre de domaines pathologiques :
- Agonists and antagonists of substance P, A.S. Dutta Drugs of the futur, 12 (8), 782 (1987);
- Substance P and pain : an updating, J.L. Henry, TINS, 3(4), 97 (1980);
- Substance P in inflammatory reactions and pain, S. Rosell, Actual. Chim. Ther., 12ème série, 249 (1985)
- Effects of Neuropeptides on Production of Inflammatory Cytokines by Human Monocytes, M. Lotz et coll., Science, 241, 1218 (1988).
- Neuropeptides and the pathogenesis of allergy, Allergy, 42, 1 à 11 (1987);
- Substance P in Human Essential Hypertension, J. Cardiocascular Pharmacology, 10 (suppl. 12), 5172 (1987).

Il a notamment été mis en évidence, par l'étude de plusieurs produits que les nouveaux dérivés de l'isoindole manifestent une activité analgésique dans la technique de Siegmund E. et coll., Proc. Soc. Exp. Biol. Med., 95, 729 (1957).

| Produit de formule générale (I) | DE 50 mg/kg p.o. |
|---|---|
| Exemple 1 | 3 |
| Exemple 2 | 2 |
| Exemple 7 | 1,5 |

L'étude de plusieurs dérivés de l'isoindole de formule générale (I) dans la technique de A. Saria et coll., Arch. Pharmacol.,324, 212-218 (1983) adaptée à la souris a permis de mettre en évidence un effet inhibiteur de l'augmentation de la perméabilité capillaire entraîné par le septide (agoniste de la substance P, ce qui témoigne d'une activité anti-inflammatoire :

| Produit de formule générale (I) | DE₅₀ mg/kg s.c. |
|---|---|
| Exemple 1 | 0,70 |
| Exemple 2 | 0,30 |
| Exemple 7 | 0,32 |

L'injection de substance P chez l'animal provoque une hypotension. Les produits étudiés dans la technique de C.A. Maggi et coll., J. Auton. Pharmac., 7, 11-32 (1987) manifestent un effet antagoniste chez le rat vis-à-vis de cette hypotension. Notamment les produits administrés à la dose de 1 mg/kg i.v./mn, pendant 5 mn, provoquent un antagonisme de l'hypotension induite par une injection i.v. de 250 ng/kg de substance P.

| Produit de formule générale (I) | % d'inhibition de l'hypotension |
|---|---|
| Exemple 1 | 73 |
| Exemple 2 | 68 |

Par ailleurs, les dérivés de l'isoindole selon la présente invention ne présentent pas de toxicité, ils se sont montrés atoxiques chez la souris par voie sous cutanée à la dose de de 40 mg/kg ou par voie orale à la dose de 100 mg/kg.

D'un intérêt particulier sont les produits de formule générale (I) dans laquelle :
- les radicaux R sont des atomes d'hydrogène,
- les symboles R' sont des radicaux phényle,
- le symbole X représente un atome d'oxygène, ou un radical NH,
- le symbole R₁ représente un radical phényle éventuellement substitué par un radical alcoyloxy pouvant être éventuellement substitué [par un radical dialcoylamino ou dialcoylamino dont les parties alcoyle forment avec l'atome d'azote auquel elles sont rattachées, un hétérocycle à 5 à 6 chaînons], ou substitué par un radical dialcoylamino dont les parties alcoyle peuvent former avec l'atome d'azote auquel elles sont rattachées, un hétérocycle tel que défini ci-dessus,
- le symbole R₂ représente un atome d'hydrogène ou un radical alcoyle,
- le symbole R₃ représente un atome de fluor ou de chlore ou un radical hydroxy et
- le symbole R₄ représente un atome d'hydrogène ou simultanément à R₃ représente un atome de fluor.

Et parmi ces produits plus spécialement intéressants sont les produits suivants :
- {{[(pyrrolidinyl-1)-3 propoxy-2]phényl}acétyl}-2 diphényl-4,4 fluoro-7 perhydroisoindole,
- diphényl-4,4 fluoro-7 [(méthoxy-2 phényl)-2 propionyl-(S)]-2 perhydroisoindole,
- [(diméthylamino-3 propoxy)-2 phényl]acétyl-2 diphényl-4,4 fluoro-7 perhydroisoindole,
- diphényl-7,7 [(méthoxy-2 phényl)-2 propionyl-(S)]-2 perhydroisoindolol-4,
- [(méthoxy-2 phényl)acétyl]-2 diphényl-4,4 fluoro-7 perhydroisoindole,

sous leurs formes stéréoisomères ainsi que leurs mélanges et le cas échéant leurs sels.

Les exemples suivants donnés à titre non limitatif illustrent la présente invention.

Dans les exemples qui suivent, il est entendu que, sauf mention spéciale, les spectres de RMN du proton ont été faits à 250 MHz dans le diméthylsulfoxyde; les déplacements chimiques sont exprimés en ppm.

### EXEMPLE 1

A une solution de 0,72 g de chlorhydrate de diphényl-4,4 fluoro-7 perhydrcisoindole-(3aR,7R,7aR), 0,5 g d'acide (diméthylamino-3 propoxy)-2 phénylacétique, 0,03 g d'hydroxy-1 benzotriazole dans 75 cm³ de dichlorométhane refroidie à + 4°C on ajoute en 10 minutes une soluticn de 0,5 g de (diméthylamino-3 propyl)-1 éthyl-3 carbodiimide dans 50 cm³ de dichlorométhane sec puis 0,37 cm³ de diisopropyléthylamine. Le mélange réactionnel est agité 3 heures à 0°C puis lavé 2 fois par 50 cm³ d'eau et 2 fois par 50 cm³ de solution saturée de chlorure de sodium. La phase organique est séchée sur sulfate de magnésium et concentrée à sec sous pression réduite (2,7 kPa). Le résidu est repris par 21 cm³ d'acide chlorhydrique 0,1 N, 50 cm³ d'éther diéthylique et 30 cm³ d'eau. La phase aqueuse est séparée et lyophilisée pour donner 0,85 g de chlorhydrate de [(diméthylamino-3 propoxy)-2 phényl]acétyl-2 diphényl-4,4 fluoro-7 perhydroisoindole-(3aR,7R,7aR), sous la forme d'un lyophilisat blanc.

Spectre infra-rouge (KBr, bandes caractéristiques, cm⁻¹): 3060, 3030, 2960, 2890, 2800, 2200, 1635, 1605, 1495, 1460, 1445, 1250, 755, 705.

Spectre RMN du proton (DMSO-d₆) (à température ambiante, on observe le mélange des deux rotamères):
0,95-1,35 et 1,8-2,1 (2mt, 2x1H, CH₂ en 6) ; 2,6-2,8 (mt, 6H, N(CH₃)₂); 3,9 et 4,05 (2mt, 2x1H, OCH₂) ; 4,8 et 4,85 (2d larges, J-50, 1H, CHF) ; 6,8 à 7,5 (mt, 14H, aromatiques).

Le chlorhydrate de diphényl-4,4 fluoro-7 perhydroisoindole-(3aR,7R,7aR) peut être obtenu de la manière suivante :

Une solution de 2,25 g de t-butoxycarbonyl-2 diphényl-4,4 fluoro-7 perhydroisoindole-(3aR,7R,7aR) dans 25 cm³ de dioxanne est traitée par une solution 5,8 N d'acide chlorhydrique dans le dioxanne et agitée 2 heures à 20°C puis concentrée à sec sous pression réduite (2,7 kPa). Le résidu est concrèté par addition de 100 cm³ d'oxyde d'isopropyle, le solide est filtré et séché pour donner 1,8 g de chlorhydrate de diphényl-4,4 fluoro-7 perhydroisoindole-(3aR,7R,7aR) sous la forme d'une poudre crème.

Spectre RMN du proton (DMSO-d₆): 1,0-1,35 (mt, 1H du CH₂en 6) ; 4,9 (d large, J=50, 1H, CHF) ; 7,1 à 7,5 (mt, 14H, aromatiques) ; 9,05 et 9,9 (2 mf, 2x1H, NH₂ ⁺).

Le t-butoxycarbonyl-2 diphényl-4,4 fluoro-7 perhydroisoindole-(3aR,7R,7aR) peut être obtenu de la manière suivante :

Une solution de 4,87 g de t-butoxycarbonyl-2 diphényl-4,4 trifluorométhylsulfonyloxy-7 perhydroisoindole- (3aR,7S,7aR) dans 150 cm³ de dichlorométhane sec est traitée par 22,6 cm³ de solution 1M de fluorure de tétrabutylammonium dans le tétrahydrofuranne puis agitée 17 heures à 20°C et concentré à sec sous pression réduite (2,7 kPa). Le résidu est chromatographié sur une colonne de gel de silice (granulométrie 0,2-0,063 mm, diamètre 4,5 cm, hauteur 35 cm) en éluant sous une pression de 0,4 bar d'azote par un mélange de cyclohexane et d'acétate d'éthyle (75/25) et en recueillant des fractions de 20 cm³. Les fractions 28 à 38 sont réunies et concentrées à sec sous pression réduite (2,7 kPa) pour donner 1,44 g de t-butoxycarbonyl-2 diphényl-4,4 fluoro-7 perhydroisoindole-(3aR,7R,7aR) sous la forme de cristaux blancs fondant à 200°C, [α]_{D} ²⁰ = - 225° (c=1, CHCl₃).

Le t-butoxycarbonyl-2 diphényl-4,4 trifluorométhylsulfonyloxy-7 perhydroisoindole-(3aR,7S,7aR) peut être obtenu de la manière suivante :

A une solution de 6,7 g de t-butoxycarbonyl-2 diphényl-7,7 perhydroisoindolol-4-(3aR,4S,7aR) dans 100 cm³ de dichlorométhane sec refroidie à -30°C on ajoute 1,5 cm³ de pyridine puis, en 10 minutes, une solution de 3,2 g d'anhydride trifluorométhanesulfonique dans 25 cm³ de dichlorométhane sec. Le mélange réactionnel est agité 2 heures à -30°C puis dilué par 250 cm³ d'eau et 100 cm³ de dichlorométhane. La phase organique est lavée par 200 cm³ de solution saturée de bicarbonate de sodium et par 200 cm³ de solution saturée de chlorure de sodium puis séchée et concentrée à sec sous pression réduite (2,7 kPa) pour donner 8,6 g de t-butoxycarbonyl-2 diphényl-4,4 trifluorométhylsulfonyloxy-7 perhydroisoindole-(3aR,7S,7aR) sous la forme d'une meringue jaune utilisée telle quelle dans la suite de la synthèse.

Le t-butoxycarbonyl-2 diphényl-7,7 perhydroisoindolol-4-(3aR,4S,7aR) peut être obtenu de la manière suivante :

A une solution de 13 g de diphényl-7,7 perhydroisoindolol-4-(3aR,4S,7aR) et 0,5 g de diméthylamino-4 pyridine dans 450 cm³ de dichlorométhane on ajoute 10,55 g de diterbutyldicarbonate. Après agitation durant 2 heures à 25°C, le mélange réactionnel est concentré à sec sous pression réduite (2,7 kPa) et le résidu est cristallisé dans 50 cm³ d'éther éthylique. On obtient 9 g de t-butoxycarbonyl-2 diphényl-7,7 perhydroisoindolol-4-(3aR,4S,7aR) sous la forme de cristaux blancs fondant à 190°C.

Le diphényl-7,7 perhydroisoindolol-4-(3aR,4S,7aR) peut être obtenu de la manière suivante :

A une solution de 100 g de chlorhydrate de diphényl-7,7 perhydroisoindolone-4-(3aR,7aR) dans 1000 cm³ de méthanol absolu refroidie à 5°C on ajoute en 90 minutes une solution de 7,18 g de borohydrure de sodium dans 500 cm³ de méthanol additionnée de 20 gouttes de solution concentrée de soude. Après agitation pendant 2,5 heures entre 5 et 10°C, les cristaux formés sont essorés et repris par 900 cm³ d'eau et 1000 cm³ d'éther éthylique. La solution est filtrée et alcalinisée par 15 cm³ de solution de soude 4N puis agitée 2 h à 5°C. Les cristaux formés sont essorés, lavés à l'éther éthylique et séchés pour donner 28,8 g de diphényl-7,7 perhydroisoindolol-4-(3aR,7S,7aR) sous la forme de cristaux blancs fondant à 205°C, [α]_{D} ²⁰ = - 230° (c=1, CHCl₃).

Le chlorhydrate de diphényl-7,7 perhydroisoindolone-4-(3aR,7aR) peut être préparé de la manière suivante :

A une suspension de 200 g de chlorhydrate de diphényl-7,7 perhydroisoindolone-4 (3aRS,7aRS) dans 2000 cm³ d'acétate d'éthyle, on ajoute lentement, sous agitation, 500 cm³ de soude aqueuse 4N ; l'agitation est poursuivie jusqu'à dissolution du produit de départ. La solution organique est lavée par 250 cm³ d'eau distillée, par 250 cm³ d'une solution aqueuse saturée en chlorure de sodium, séchée sur sulfate de magnésium et filtrée. A la solution ainsi obtenue, on ajoute, sous agitation, une solution de 92,8 g d'acide L-(+) mandélique dans 1000 cm³ d'acétate d'éthyle ; après 4 heures d'agitation, les cristaux obtenus sont essorés, lavés par 250 cm³ d'acétate d'éthyle (2 fois) et séchés. Les cristaux sont repris par 2000 cm³ d'eau distillée ; le mélange est chauffé au reflux, sous agitation, pendant 15 minutes ; les cristaux insolubles sont essorés, lavés par 100 cm³ d'eau distillée (2 fois) et séchés. Ils sont recristallisés dans un mélange de 1100 cm³ d'acétonitrile et de 500 cm³ d'eau distillée; les cristaux obtenus sont essorés, lavés par 40 cm³ d'acétonitrile (3 fois) et séchés. On obtient 80 g de (L)-mandélate de diphényl-7,7 perhydroisoindolone-4 (3aR,7aR); [α]_{D} ²⁰ = -164° (c=1, méthanol).

A 80 g de (L)-mandélate de diphényl-7,7 perhydroisoindolone-4 (3aR,7aR), on ajoute 400 cm³ de soude aqueuse 1N et 600 cm³ d'acétate d'éthyle ; le mélange est agité à température ambiante jusqu'à dissolution du produit de départ ; la solution organique est lavée par 250 cm³ d'eau distillée, par 250 cm³ d'une solution aqueuse saturée en chlorure de sodium, séchée sur sulfate de magnésium et filtrée ; elle est acidifiée, sous agitation, par addition de 30 cm³ d'acide chlorhydrique 9N ; les cristaux obtenus sont essorés, lavés par 50 cm³d'acétate d'éthyle (2 fois), par 50 cm³ d'oxyde d'isopropyle et séchés. On obtient 52,3 g de chlorhydrate de diphényl-7,7 perhydroisoindolone-4 (3aR,7aR), fondant à 270°C avec décomposition; [α]_{D} ²⁰ = -282° (c-0,5, méthanol).

Le chlorhydrate de diphényl-7,7 perhydroisoindolone-4-(3aRS,7aRS) peut être préparé selon la méthode suivante :

A 15 g de palladium sur charbon à 10%, on ajoute 150 g de benzyl-2 diphényl-7,7 perhydroisoindolone-4 (3aRS,7aRS), 1500 cm³ de méthanol et 450 cm³ d'acide chlorhydrique 1N ; le mélange réactionnel est hydrogéné, sous agitation, à température ambiante et sous pression atmosphérique. Après 5 heures de réaction le volume théorique d'hydrogène a été absorbé ; le mélange réactionnel est filtré, puis concentré à sec sous pression réduite (2,7 kPa) ; le résidu est cristallisé dans 200 cm³ d'éthanol ; les cristaux obtenus sont essorés, lavés par 50 cm³d'éthanol et séchés. On obtient 110 g de chlorhydrate de diphényl-7,7 perhydroisoindolone-4-(3aRS,7aRS) fondant à 270°C avec décomposition.

Spectre de RMN du proton : 2,03 (Mt, 1H, 1H du H en 5 ou 6) ; 2,3 (Mt, 1H, 1H du - H en 5 ou 6) ; 2,48 (DD, en partie masqué, 1H du -CH₂- en 1) ; 2,69 (DD, 1H, 1H du -CH₂- en 1); 2,8 (Mt, 2H, -CH₂- en 6 ou 5) ; 3,34 (DD, en partie masqué, 1H du -CH₂- en 3) ; 3,5 (Mt, 1H, -CH- en 3a) ; 3,82 (DD, 1H, 1H du -CH₂- en 3) ; 3,95 (Mt, 1H, -CH- en 7a) ; 7,15 à 7,65 (Mt, 10H, aromatiques) ; 9,43 (Mf, 2H, -NH₂-Cl).

Spectre Infra-rouge (KBr) bandes caractéristiques en cm⁻¹ : 3600-3300, 3100-3000, 3000-2850, 3100-2400, 1715, 1595, 1580, 1495, 1470, 1445, 775, 750, 705.

La benzyl-2 diphényl-7,7 perhydroisoindolone-4-(3aRS,7aRS) peut être préparée de la manière suivante :

A une solution de 155 g de diphényl-4,4 cyclohexène-2 one-1 et de 202 cm³ de N-butoxyméthyl N-triméthylsilylméthyl benzylamine dans 1000 cm³ de dichlorométhane sec, on ajoute 5 gouttes d'acide trifluoroacétique et chauffe le mélange réactionnel au reflux pendant 45 minutes. On ajoute 50 cm³ de N-butoxyméthyl N-triméthylsilylméthyl benzylamine et 3 gouttes d'acide trifluoroacétique et agite encore 45 minutes au reflux avant d'ajouter de nouveau 25 cm³ de N-butoxyméthyl N-triméthylsilylméthyl benzylamine et 3 gouttes d'acide trifluoroacétique. Le mélange réactionnel est agité au reflux 45 minutes puis traité par 50 g de carbonate de potassium, filtré et concentré à sec sous pression réduite (2,7 kPa). Le résidu est dissous dans 200 cm³ d'oxyde d'isopropyle et la solution refroidie à 0°C pendant 1 heure. Les cristaux sont essorés, lavés 2 fois par 15 cm³ d'oxyde d'isopropyle et séchés pour donner 193 g de benzyl-2 diphényl-7,7 perhydroisoindolone-4-(3aRS,7aRS) sous la forme de cristaux blancs fondant à 132°C.

La N-butoxyméthyl N-triméthylsilylméthyl benzylamine peut être préparée selon la méthode de Y. Terao et coll., Chem. Pharm. Bull., 33, 2762 (1985).

### EXEMPLE 2

A une solution refroidie à +5°C de 1 g de chlorhydrate de diphényl-4,4 fluoro-7 perhydroisoindole-(3aR,7R,7aR) et de 0,924 g d'acide {[(pyrrolidinyl-1)-3 propoxy-2]phényl}-2 acétique dans 40 cm³ de dichlorométhane sec, on ajoute 0,04 g d'hydrate d'hydroxybenzotriazole, puis 0,79 g chlorhydrate de (diméthylamino-3 propyl)-1 éthyl-3 carbodiimide et 0,51 cm³ de diisopropyléthylamine. Après 2,5 heures d'agitation à +5°C et 20 heures à 20°C, le mélange réactionnel est lavé 2 fois par 50 cm³ d'eau, séché sur sulfate de magnésium, puis concentré à sec à 40°C sous pression réduite (2,7 kPa). Le résidu est chromatographié sur colonne de gel de silice (granulométrie 0,04-0,06 mm, diamètre 2,4 cm, hauteur 31 cm), en éluant sous une pression de 0,5 bar d'azote par un mélange d'acétate d'éthyle, d'acide acétique et d'eau (60/10/10 en volumes) et en recueillant des fractions de 25 cm³. Les fractions 11 à 31 sont réunies et concentrées à sec sous pression réduite (2,7 kPa). Le résidu est repris avec 20 cm³ de dichlorométhane, la solution est lavée par 20 cm³ de solution aqueuse d'hydroxyde de sodium 1N, puis est séchée sur sulfate de magnésium, et concentrée à sec. Ce lavage basique est effectué une nouvelle fois. On obtient 0,68 g de {{[(pyrrolidinyl-1)-3 propoxy-2]phényl}acétyl}-2 diphényl-4,4 fluoro-7 perhydroisoindole-(3aR,7R,7aR) sous la forme d'un solide blanc.

Spectre Infra-rouge (KBr, bandes caractéristiques, cm⁻¹) : 3085, 3055, 3035, 2950, 2875, 2785, 1640, 1600, 1495, 1455, 1440, 1245, 750, 700.

Spectre RMN du proton (DMSO-d₆ + CF₃COOD): 1,1-1,45 (mt, 1H, 1H en 6); 1,9 (mt, 4H, 2CH₂ en 3 et 4 du pyrolidino); 2,27 (mt, 1H, 1H en 5); 3,77 (d, J=10, 1H, H en 1); 4,03 (mt, 2H, OCH₂); 4,78 (d large, J=50, 1H, CHF); 7,1 à 7,5 (mt, 14H, aromatiques).

Le chlorhydrate de l'acide {[(pyrrolidinyl-1)-3 propoxy-2]phényl} acétique peut être préparé de la façon suivante:

A 24,9 g de {[(pyrrolidinyl-1)-3 propoxy-2]phényl}acétate de méthyle on ajoute 700 cm³ d'une solution d'acide chlorhydrique 6N. Après 2 heures de reflux la solution est concentrée à sec à 50°C sous pression réduite. Le résidu est ensuite repris par 100 cm³ de toluène puis la solution est concentrée à sec sous pression réduite. Après un traitement identique avec du toluène, on obtient 21,9 g de chlorhydrate de l'acide {[(pyrrolidinyl-1)-3 propoxy-2]phényl} acétique, sous la forme d'un solide beige, fondant à 173°C.

Spectre RMN du proton (250 MHz, DMSO-d₆) : 1,8 à 2,1 (mt, 4H, -N(CH₂CH₂)₂; 2,15 (mt, 2H, CH₂-CH₂-CH₂); 2,98 et 3,52 (2mt, 2x2H, -N(CH₂CH₂)₂); 3,26 (mt, 2H, CH₂-N<); 3,5 (2s, 2H, CH₂COO); 4,06 (t, J=6, 2H, OCH₂); 6,32 (mt, 2H, aromatiques en 3 et 5); 7,23 (mt, 2H, aromatiques en 4 et 6); 11,2 (mf, 1H, -NH⁺<).

Le {[(pyrrolidinyl-1)-3 propoxy-2]phényl} acétate de méthyle peut être préparé de la façon suivante:

A une solution de 36 g de (bromo-3 propoxy)-2 phényl acétate de méthyle dans 400 cm³ d'acétonitrile, on ajoute successivement 18,4 g de carbonate de potassium, 4,5 g d'iodure de sodium, puis 9,54 g de pyrrolidine. Le mélange réactionnel est porté au reflux pendant 5 heures puis agité à 20°C pendant 20 heures et dilué avec 800 cm³ de dichlorométhane. La solution est lavée par 80 cm³ d'eau puis par deux fois 80 cm³ de saumure. La phase chlorométhylénique est séchée sur sulfate de sodium, concentrée à sec sous pression réduite. Le résidu est chromatographié sur une colonne de gel de silice (granulométrie 0,04-0,06 mm, diamètre 4,5 cm, hauteur 48 cm), en éluant sous une pression de 0,4 bar d'azote par un mélange d'acétate d'éthyle et de méthanol (90/10 en volumes) et en recueillant des fractions de 500 cm³. Les fractions 3 à 8 sont réunies et concentrées à sec à 50°C sous pression réduite (2,7 kPa). on obtient 24,9 g de {[(pyrrolidinyl-1)-3 propoxy-2]phényl}acétate de méthyle, sous la forme d'une huile orangée.

Spectre infra-rouge (CCl₄, bandes caractiques, cm⁻¹): 3065, 3030, 2950, 2930, 2880, 2790, 1740, 1600, 1585, 1490, 1770, 1455, 1430, 1250.

Le (bromo-3 propoxy)-2 phényl acétate de méthyle peut être préparé de la façon suivante:

A une solution de 153 g d'(hydroxy-2 phényl) acétate de méthyle dans 1400 cm³ d'acétonitrile, on ajoute 254,5 g de carbonate de potassium et 654 cm³ de dibromo-1,3 propane. La suspension obtenue est portée au reflux pendant 20 heures, refroidie à 20°C, puis filtrée. Le filtrat est concentré à sec à 50 C sous pression réduite. Le résidu est chromatographié sur une colonne de gel de silice (granulométrie 0,04-0,06 mm, diamètre 8,8 cm, hauteur 43 cm) en éluant sous une pression d'azote de 0,5 bar par un mélange de cyclohexane et d'acétate d'éthyle (95/5 en volumes) et en recueillant des fractions de 300 cm³. Les fractions 18 à 53 sont réunies et concentrées à sec sous pression réduite (2,7 kPa). On obtient 130,6 g de (bromo-3 propoxy)-2 phényl acétate de méthyle, sous la forme d'une huile orange.

Spectre RMN du proton (250 MHz, CDCl₃) : 2,32 (qt, J=6,5, 2H, CH₂-CH₂-CH₂); 3,26 (t, J=6,5, 2H, CH₂Br); 3,65 (s, 2H, COOCH₃); 3,7 (s, 2H, CH₂COO); 4,13 (t, J=6,5, 2H, OCH₂); 6,94 (mt, 2H, aromatiques en 3 et 5); 7,25 (mt, 2H, aromatiques en 4 et 6).

### EXEMPLE 3

En opérant comme à l'exemple 9 ci-après, à partir de 0,16 g d'acide diméthylamino-2 phénylacétique et de 0,30 g de chlorhydrate de diphényl-4,4 fluoro-7 perhydroisoindole- (3aR,7S,7aR) on obtient 0,11 g de [(diméthylamino-2 phényl)acétyl]-2 diphényl-4,4 fluoro-7 perhydroisoindole- (3aR,7S,7aR) sous la forme d'une meringue blanche.

Spectre infra-rouge (KBr, bandes caractéristiques, cm⁻¹): 3090, 3060, 3030, 2940, 2875, 2825, 2770, 1645, 1595, 1580, 1495, 1450, 1420, 755, 730, 700.

Spectre RMN du proton (à température ambiante, on observe le mélange de deux rotamères) : 2,35 et 2,58 (2s, 6H, N(CH₃)₂), 4,2-4,6 (mt, 1H, CHF), 6,9-7,5 (mt, 14H, aromatiques).

Le chlorhydrate de diphényl-4,4 fluoro-7 perhydroisoindole-(3aR,7S,7aR) peut être préparé de la façon suivante:

En opérant comme à l'exemple 8 ci-après à partir de 0,5 g de tertiobutyloxycarbonyl-2 diphényl-4,4 fluoro-7 perhydroisoindole-(3aR,7S,7aR), on obtient 0,35 g de chlorhydrate de diphényl-4,4 fluoro-7 perhydroisoindole-(3aR,7S,7aR) sous la forme d'un solide gris.

Spectre infra-rouge (KBr, bandes caractéristiques, cm⁻¹): 3420, 3090, 3050, 3025, 2970, 2920, 2800-2250, 1590, 1580, 1495, 1460, 1445, 1060, 750, 730, 700.

Le tertiobutyloxycarbonyl-2 diphényl-4,4 fluoro-7 perhydro-isoindole-(3aR,7S,7aR) peut être préparé de la façon suivante:

A une solution refroidie à +5°C de 1,0 g de tertiobutyloxycarbonyl-2 diphényl-7,7 perhydroisoindolol-4- (3aR,4R,7aR) dans 20 cm³ de dichlorométhane sec, on ajoute une solution de 0,37 cm³ de trifluorothio-4 morpholine dans 10 cm³ de dichlorométhane sec. Après 2 heures d'agitation à +5°C, le mélange réactionnel est lavé par 20 cm³ de solution aqueuse de bicarbonate de sodium à 5% puis séché sur sulfate de magnésium et concentré à sec sous pression réduite (2,7 kPa). Le résidu est chromatographié sur une colonne de gel de silice (granulométrie 0,04 mm - 0,06 mm, diamètre 2,4 cm, hauteur 35 cm), en éluant sous une pression d'azote de 0,8 bar, par un mélange de cyclohexane et d'acétate d'éthyle (90/10 en volumes) et en recueillant des fractions de 25 cm³. Les fractions 25 à 34 sont réunies et concentrées à sec. On obtient 0,27 g de tertiobutyloxycarbonyl-2 diphényl-4,4 fluoro-7 perhydroisoindole-(3aR,75,7aR) sous la forme d'une meringue blanche.

Spectre infra-rouge (KBr, bandes caractéristiques, cm⁻¹): 3090, 3060, 3030, 2975, 2930, 2875, 1695, 1595, 1580, 1495, 1450, 1405, 1365, 1175, 755, 730, 700.

### EXEMPLE 4

A une solution de 0,57 g de bromhydrate d'acide (pyrrolidino-2) phénylacétique dans 20 cm³ de dichlorométhane sec, refroidie à 4°C, on ajoute 0,28 cm³ de triéthylamine et 0,32 g de carbonyldiimidazole. On agite une heure à + 4°C, puis on ajoute une solution de 0,67 g de chlorhydrate de diphényl-4,4 fluoro-7 perhydroisoindole-(3aR,7R,7aR) dans 20 cm³ de dichlorométhane sec et 0,28 cm³ de triéthylamine. Le mélange réactionnel est agité à température ambiante pendant 24 heures, puis dilué par 100 cm³ de dichlorométhane, lavé deux fois par 50 cm³ d'eau, séché sur sulfate de magnésium, filtré et concentré à sec sous pression réduite (2,7 kPa). Le résidu est chromatographié sur colonne de gel de silice (granulométrie 0,04-0,06 mm, diamètre 3,5 cm, hauteur 38 cm), en éluant sous une pression de 0,5 bar d'azote par un mélange de cyclohexane et d'acétate d'éthyle (70/30 en volumes) et en recueillant des fractions de 20 cm³. Les fractions 26 à 54 sont réunies et concentrées à sec sous pression réduite (2,7 kPa). Le résidu est cristallisé dans un mélange d'acétonitrile et d'oxyde de diisopropyle (25/75 en volumes). On obtient 0,16 g de diphényl-4,4 fluoro-7 [(pyrrolidino-2 phényl)-acétyl]-2 perhydroisoindole-(3aR,7R,7aR) sous forme de cristaux blancs fondant à 170°C.

### _{EXEMPLE 5}

A une solution de 0,19 g d'acide (diméthylamino-2 phényl)-acétique dans 15 cm³ de dichlorométhane sec, refroidie à + 4°C, on ajoute 0,17 g de carbonyldiimidazole. On agite une heure à + 4°C, puis on ajoute une solution de 0,35 g de chlorhydrate de chloro-7 diphényl-4,4 perhydroisoindole-(3aR,7R,7aR) dans 10 cm³ de di-chlorométhane sec, puis une solution de 0,15 cm³ de triéthylamine dans 10 cm³ de dichlorométhane sec. Le mélange réactionnel est agité à température ambiante pendant 20 heures, puis dilué par 120 cm³ de dichlorométhane, lavé par 80 cm³ d'eau, puis par une solution aqueuse saturée en chlorure de sodium, séché sur sulfate de magnésium,filtré et concentré à sec sous pression réduite (2,7 kPa). Le résidu est chromatographié sur colonne de gel de silice (granulométrie 0,04-0,06 mm, diamètre 2 cm, hauteur 22 cm), en éluant sous une pression de 0,4 bar d'azote par un mélange d'acétate d'éthyle et de cyclohexane (75/25 en volumes) et en recueillant des fractions de 20 cm³. Les fractions 6 à 9 sont réunies et concentrées à sec sous pression réduite (2,7 kPa). Le produit, obtenu sous forme de base, est converti en chlorhydrate par dissolution dans 25 cm³ d'éther éthylique,puis ajout de 5 cm³ d'une solution 3,2 N d'acide chlorhydrique dans l'éther éthylique, lavage à l'éther éthylique et séchage. On obtient 0,14 g de chlorhydrate de chloro-7 [(diméthylamino-2 phényl)-acétyl]-2 diphényl-4,4 perhydroisoindole-(3aR,7R,7aR) sous la forme de cristaux blancs fondant à 190°C.

Le chlorhydrate de chloro-7 diphényl-4,4 perhydro-isoindole-(3aR,7R,7aR) peut être obtenu de la manière suivante :

Une solution de 0,4 g de chloro-7 chlorocarbonyl-2 diphényl-4,4 perhydroisoindole-(3aR,7R,7aR) dans 6 cm³ d'une solution aqueuse 1N d'acide chlorhydrique et 14 cm³ de tétrahydrofuranne est portée à 80°C sous agitation pendant 9 heures. Le mélange réactionnel est concentré à sec sous pression réduite (2,7 kPa). On obtient 0,35 g de chlorhydrate de chloro-7 diphényl-4,4 perhydroisoindole- (3aR,7R,7aR) sous forme de solide blanc.

Spectre infra-rouge (KBr, bandes caractéristiques, cm⁻¹): 3055, 3025, 3000, 2250, 1600, 1495, 1580, 1460, 1445, 1435, 760, 750, 735, 700.

Le chloro-7 chlorocarbonyl-2 diphényl-4,4 perhydroisoindole-(3aR,7R,7aR) peut être obtenu de la manière suivante :

A une solution refroidie à + 4°C de 1 g de diphényl-7,7 tert-butyloxycarbonyl-2 perhydroisoindolol-4-(3aR,4S,7aR) dans 60 cm³de chloroforme, on ajoute successivement 1,3 g de carbonate de calcium, puis 2 g de pentachlorure de phosphore et on agite à température ambiante pendant 20 heures. Le mélange réactionnel est ensuite filtré, dilué par 80 cm³ de chloroforme, lavé deux fois par 80 cm³ d'eau, séchée sur sulfate de magnésium et concentré à sec sous pression réduite (2,7 kPa). Le résidu est chromatographié sur colonne de gel de silice (granulométrie 0,04-0,06 mm, diamètre 2,5 cm, hauteur 34 cm), en éluant sous une pression de 0,4 bar d'azote par un mélange de cyclohexane et d'acétate d'éthyle (30/70 en volumes) et en recueillant des fractions de 20 cm³˙ Les fractions 7 à 10 sont réunies et concentrées à sec sous pression réduite (2,7 kPa). On obtient 0,44 g de chloro-7 chlorocarbonyl-2 diphényl-4,4 perhydroisoindole-(3aR,7R,7aR) sous la forme d'un solide blanc.

Spectre infra-rouge (Solution CCl₄, bandes caractéristiques, cm⁻¹): 3090, 3065, 3035, 2930, 2855, 1745, 1600, 1585, 1495, 1450, 700.

### EXEMPLE 6

A une solution de 0,43 g d'acide (diméthylamino-2 phényl) acétique dans 15 cm³ de dichlorométhane sec, refroidie à + 4°C, on ajoute 0,39 g de carbonyldiimidazole. On agite une heure à + 4°C, puis on ajoute une solution de 0,84 g de chlorhydrate de chloro-7 diphényl-4,4 perhydroisoindole-(3aR,7S,7aR) dans 10 cm³ de dichlorométhane sec, puis une solution de 0,34 cm³ de triéthylamine dans 10 cm³ de dichlorométhane sec. Le mélange réactionnel est agité à température ambiante pendant 20 heures, puis dilué par 100 cm³ de dichlorométhane, lavé par 50 cm³ d'eau, puis par une solution aqueuse saturée en chlorure de sodium, séché sur sulfate de magnésium, filtré et concentré à sec sous pression réduite (2,7 kPa). Le résidu est chromatographié sur colonne de gel de silice (granulométrie 0,04-0,06 mm, diamètre 3 cm, hauteur 23 cm), en éluant sous une pression de 0,4 bar d'azote par un mélange d'acétate d'éthyle et de cyclohexane (25/75 en volumes) et en recueillant des fractions de 80 cm³. La fraction 2 est concentrée à sec sous pression réduite (2,7 kPa). Le produit obtenu sous forme de base, est converti en chlorhydrate par dissolution dans 4 cm³ d'acétonitrile, puis ajout de 6 cm³ d'une solution 3,2 N d'acide chlorhydrique dans l'éther éthylique, lavage à l'éther isopropylique et séchage. On obtient 0,08 g de chlorhydrate de chloro-7 [(diméthylamino-2 phényl)-acétyl]-2 diphényl-4,4 perhydroisoindole-(3aR,7S,7aR) sous la forme d'un solide beige.

Spectre infra-rouge (KBr, bandes caractéristiques, cm⁻¹): 3055, 3025, 2950, 1635, 1490, 1460, 1440, 760, 750, 700.

Spectre RMN du proton (DMSO-d₆) (à 403°K on observe le mélange des deux rotamères, DMSO-d₆ + CF₃COOD, signaux principaux): 3 et 3,13 (2s, 6H, N(CH₃)₂) ; 4,54 et 4,63 (2mt, 1H, CHCl) ; 7 à 7,8 (mt, 14H, aromatiques).

Le chlorhydrate de chloro-7 diphényl-4,4 perhydroisoindole-(3aR,7S,7aR) peut être obtenu de la manière suivante :

A une solution de 1,03 g de tert-butyloxycarbonyl-2 chloro-7 diphényl-4,4 perhydroisoindole-(3aR,7S,7aR) dans 5 cm³ de dioxanne on ajoute 10 cm³ d'une solution 6,3 N d'acide chlorhydrique dans le dioxanne. Le mélange réactionnel est agité à température ambiante pendant 2 heures, puis concentré à sec sous pression réduite (2,7 kPa). On obtient 0,84 g de chlorhydrate de chloro-7 diphényl-4,4 perhydroisoindole-(3aR,7S,7aR) sous forme d'un solide, utilisé brut dans l'essai suivant.

Le tert-butyloxycarbonyl-2 chloro-7 diphényl-4,4 perhydro-isoindole-(3aR,7S,7aR) peut être obtenu de la manière suivante :

Une solution de 1 g de tert-butyloxycarbonyl-2 diphényl-7,7 perhydroisoindolol-4-(3aR,4S,7aR) dans 10 cm³ de chlorure de thionyle est agitée 3 heures à 80°C. Le mélange réactionnel est ensuite concentré à sec sous pression réduite (2,7 kPa). On obtient 1,03 g de tert-butyloxycarbonyl-2 chloro-7 diphényl-4,4 perhydroisoindole-(3aR,7S,7aR) sous la forme d'un solide, utilisé brut dans l'essai suivant.

### EXEMPLE 7

A une solution de 0,36 g d'acide (méthoxy-2 phényl)-2 propionique-(S) dans 20 cm³ de dichlorométhane sec, refroidie à + 4°C, on ajoute 0,32 g de carbonyldiimidazole. On agite une heure à + 4°C, puis on ajoute une solution de 0,67 g de chlorhydrate de diphényl-4,4 fluoro-7 perhydroisoindole-(3aR,7R,7aR) dans 20 cm³ de dichlorométhane sec et 0,28 cm³ de triéthylamine. Le mélange réactionnel est agité à température ambiante pendant 20 heures, dilué dans 200 cm³ de dichlorométhane puis lavé par 50 cm³ d'eau, séché sur sulfate de magnésium, filtré et concentré à sec sous pression réduite (2,7 kPa).

Le résidu est chromatographié sur colonne de gel de silice (granulométrie 0,04-0,06 mm diamètre 3 cm, hauteur 20 cm), en éluant sous une pression de 0,4 bar d'azote par un mélange d'acétate d'éthyle et de cyclohexane (60/40 en volumes) et en recueillant des fractions de 20 cm³. Les fractions 10 à 15 sont réunies et concentrées à sec sous pression réduite (2,7 kPa). Le résidu est cristallisé dans 0,6 cm³ d'oxyde d'isopropyle. Les cristaux obtenus sont essorés, lavés par de l'oxyde d'isopropyle, puis séchés. On obtient 0,19 g de diphényl-4,4 fluoro-7 [(méthoxy-2 phényl)-2 propionyl-(S)]-2 perhydroisoindole-(3aR,7R,7aR) sous forme de cristaux blancs fondant à 195°C.

Le chlorhydrate de diphényl-4,4 fluoro-7 perhydroisoindole-(3aR,7R,7aR) peut être obtenu de la manière suivante :

A une solution de 3,7 g de diphényl-4,4 fluoro-7 tert-butyloxycarbonyl-2 perhydroisoindole-(3aR,7R,7aR) dans 40 cm³ de dioxanne on ajoute une solution de 40 cm³ de dioxanne chlorhydrique 6,3 N et on agite à température ambiante pendant 2 heures. Le mélange réactionnel est concentré à sec sous pression réduite (2,7 kPa), trituré dans de l'oxyde de diisopropyle, filtré et séché. On obtient 3,1 g de chlorhydrate de diphényl-4,4 fluoro-7 perhydroisoindole-(3aR,7R,7aR) sous forme de cristaux blancs fondant à 200°C avec décomposition .

Le diphényl-4,4 fluoro-7 tert-butyloxycarbonyl-2 perhydro-isoindole-(3aR,7R,7aR) peut être obtenu de la manière suivante :

A une solution refroidie à +5°C de 9,4 g de diphényl-7,7 tert-butyloxycarbonyl-2 perhydroisoindolol-4-(3aR,4S,7aR) dans 250 cm³ de dichlorométhane sec on ajoute une solution de 3,5 cm³ de trifluorure de morpholinosoufre dans 50 cm³ de dichlorométhane. Le mélange réactionnel est agité 4 heures à +5°C puis dilué avec 300 cm³ de dichlorométhane, lavé par 250 cm³ de solution aqueuse d'hydrogénocarbonate de sodium, séché sur sulfate de magnésium, filtré et concentré à sec sous pression réduite (2,7 kPa). Le résidu est chromatographié sur colonne de gel de silice (granulométrie 0,04-0,06 mm, diamètre 3,5 cm, hauteur 42 cm , en éluant sous une pression de 0,5 bar d'azote par un mélange de cyclohexane et d'acétate d'éthyle (90/10 en volumes) et en recueillant des fractions de 120 cm³. Les fractions 13 à 17 sont réunies et concentrées à sec sous pression réduite (2,7 kPa). Le résidu est cristallisé dans du cyclohexane. On obtient 2,55 g de diphényl-4,4 fluoro-7 tert-butyloxycarbonyl-2 perhydroisoindole- (3aR,7R,7aR) sous forme de cristaux blancs fondant à 202°C.

L'acide (méthoxy-2 phényl)-2 propionique-(S) peut être obtenu de la manière suivante :

L'acide (méthoxy-2 phényl)-2 propionique-(S) peut être préparé, par analogie avec les méthodes décrites par D.A Evans et coll., Tetrahedron, 44, 5525, (1988), selon le mode opératoire suivant:

A une solution refroidie à +5°C de 4,1 g de méthyl-4 phényl-5 [(méthoxy-2 phényl)-2-(S)-propionyl]-3 oxazolidinone-2-(4S,5S) dans 60 cm³ de tétrahydrofuranne et 30 cm³ d'eau, on ajoute 1,52 g d'hydroxyde de lithium. Le mélange réactionnel est agité 3 heures à cette tempétature, puis, après retour à température ambiante, on rajoute de l'acétate d'éthyle, on décante, la phase aqueuse est acidifiée par une solution aqueuse d'acide chlorhydrique 1N, extraite par de l'acétate d'éthyle, la phase organique est séchée sur sulfate de magnésium, et concentrée à sec sous pression réduite (2,7 kPa). Le solide obtenu est recristallisé dans l'hexane, essoré et séché. On obtient 0,4 g d'acide (méthoxy-2 phényl)-2 propionique-(S) sous la forme de cristaux blancs fondant à 102°C. [α]_{D} ²⁰ = +84,6° (c=1 ; CHCl₃).

La méthyl-4 phényl-5 [(méthoxy-2 phényl)-2-(S)-propionyl]-3 oxazolidinone-2-(4S,5S)peut être obtenue de la manière suivante :

A une solution refroidie à -50°C de 10 g de méthyl-4 phényl-5 [(méthoxy-2 phényl)-acétyl]-3 oxazolidinone-2-(4S,5S) dans 150 cm³ de tétrahydrofuranne on ajoute 19,1 g d'hexaméthyl-1,1,1,3,3,3 disilazanate de sodium, on agite 45 minutes à cette température, puis on ajoute 7,72 cm³ d'iodure de méthyle. Le mélange réactionnel est ensuite agité 15 heures à température ambiante, puis dilué par de l'acétate d'éthyle, lavé par 50 cm³ d'eau, puis par 50 cm³ d'une solution aqueuse saturée de chlorure de sodium, séché sur sulfate de magnésium et concentré à sec sous pression réduite (2,7 kPa). Le résidu obtenu est cristallisé dans l'oxyde d'isopropyle, essoré et séché. On obtient 4,2 g de méthyl-4 phényl-5 [(méthoxy-2 phényl) -2-(S)-propionyl]-3 oxazolidinone-2- (4S,5S) sous la forme d'un solide blanc.

La méthyl-4 phényl-5 (méthoxy-2 phénylacétyl)-3 oxazolidinone-2-(4S,5S) peut être obtenue de la manière suivante :

A une suspension de 1,89 d'hydrure de sodium (dispersion à 80% dans la vaseline) dans 200 cm³ de tétrahydrofuranne sec on ajoute à température ambiante 9,38 g d'acide méthoxy-2 phénylacétique. On refroidit cette suspension à -30°C, on ajoute 7,77 cm³ de chlorure de pivaloyle, puis on ajoute enfin une solution refroidie à -78°C obtenue en additionnant une solution de 35,27 cm³ de butyllithium 1,6 M dans l'hexane à une solution refroidie à -78°C de 10 g de méthyl-4 phényl-5 oxazolidinone-2-(4S,5S) dans 200 cm³ de tétrahydrofuranne sec. Le mélange réactionnel est agité 45 minutes à -30°C, puis après retour à température ambiante, on ajoute 200 cm³ d'une solution aqueuse saturée de chlorure d'ammonium, puis 500 cm³ d'acétate d'éthyle ; après décantation la phase organique est lavée deux fois par 100 cm³ d'eau, puis deux fois par 100 cm³ d'une solution aqueuse saturée de chlorure de sodium; séchée sur sulfate de magnésium et concentrée à sec sous pression réduite (2,7 kPa). Le résidu est chromatographié sur colonne de gel de silice (granulométrie 0,04-0,06 mm, diamètre 4,8 cm, hauteur 36 cm), en éluant sous une pression de 0,6 bar d'azote par un mélange de cyclohexane et d'acétate d'éthyle (85/15 puis 80/20 en volumes) et en recueillant des fractions de 50 cm³. Les fractions 14 à 31 sont réunies et concentrées à sec sous pression réduite (2,7 kPa). On obtient 13,6 g de méthyl-4 phényl-5 (méthoxy-2 phénylacétyl)-3 oxazolidinone-2-(4S,5S) sous la forme d'une huile jaune.

### EXEMPLE 8

En opérant selon le mode opératoire de l'exemple 9 ci-après à partir de 0,77 g d'acide diméthylamino-2 phénylacétique et de 1,50 g de chlorhydrate de diphényl-4,4 difluoro-7,7 perhydroisoindole-(3aRS,7aRS) on obtient 1,29 g de [(diméthylamino-2 phényl)acétyl]-2 diphényl-4,4 difluoro-7,7 perhydroisoindole- (3aRS,7aRS) sous la forme d'un solide blanc fondant à 189°C.

Le chlorhydrate de diphényl-4,4 difluoro-7,7 perhydroisoindole-(3aRS,7aRS) peut être préparé de la façon suivante:

A 1,8 g de tertiobutyloxycarbonyl-2 diphényl-4,4 difluoro-7,7 perhydroisoindole-(3aRS,7aRS) on ajoute 20 cm³ de dioxanne et 20 cm³d'acide chlorhydrique 6,3N. Après 20 heures d'agitation à température ambiante, la suspension blanche obtenue est concentrée à sec à 40°C sous pression réduite (2,7 kPa). Le résidu est lavé avec de l'oxyde de diisopropyle, le solide obtenu essoré, puis séché. On obtient 1,51 g de chlorhydrate de diphényl-4,4 difluoro-7,7 perhydroisoindole-(3aRS,7aRS) sous la forme d'un solide blanc.

Spectre Infra-rouge (KBr, bandes caractéristiques, cm⁻¹): 3090, 3050, 3025, 2965, 2935, 2900, 2800-2250, 1595, 1580, 1495, 1465, 1445, 760, 730, 700.

Spectre RMN du proton (DMSO-d₆ + CF₃COOD): 1,2-1,55 et 2,12 (2mt, 2x1H, CH₂ en 6); 3-3,3 (mt, 1H, H en 7a); 3,58 (mt, 2H, CH₂ en 1); 3,76 (mt, 1H, H en 3a); 7,1 à 7,5 (mt, 10H, aromatiques).

Le tertiobutyloxydarbonyl-2 diphényl-4,4 difluoro-7,7 perhydro-isoindole-(3aRS,7aRS) peut être préparé de la façon suivante:

A une solution de 3,4 cm³ de trifluorure de diéthylamino soufre dans 20 cm³ de dichlorométhane sec, on ajoute une solution de 5,0 g de tertiobutyloxycarbonyl-2 diphényl-7,7 perhydroisoindolone-4-(3aR S,7aRS) dans 30 cm³ de dichlorométhane sec. Après 5 heures d'agitation au reflux et 20 heures à 20°C, le mélange réactionnel est lavé par 50 cm³ de solution aqueuse saturée de bicarbonate de sodium et par 50 cm³ d'eau, puis séché sur sulfate de magnésium, et concentré à sec. Le résidu est chromatographié sur une colonne de gel de silice (granulométrie 0,04 - 0,06 mm, diamètre 2,8 cm, hauteur 35 cm), en éluant sous une pression d'azote de 0,8 bar par un mélange de cyclohexane et d'acétate d'éthyle (95/5 puis 90/10 en volumes) et en recueillant des fractions de 25 cm³. Les fractions 24 à 52 sont réunies et concentrées à sec sous pression réduite (2,7 kPa). Le résidu est cristallisé dans de l'acétate d'éthyle et de l'oxyde de diisopropyle, les cristaux sont essorés puis séchés. On obtient 1,80 g de tertiobutyloxycarbonyl-2 diphényl-4,4 difluoro-7,7 perhydroisoindole-(3aRS,7aRS) sous la forme de cristaux blancs fondant à 162°C.

### EXEMPLE 9

A une solution de 0,52 g d'acide diméthylamino-2 phénylacétique dans 20 cm³ de dichlorométhane sec, on ajoute 0,49 g de N,N'-carbonyldiimidazole. On agite 30 minutes à +5°C puis on ajoute une solution de 0,93 g de chlorhydrate de diphényl-7,7 perhydroisoindolol-4-(3aR,4S,7aR) et de 0,84 cm³ de triéthylamine dans 10 cm³de dichlorométhane. Le mélange réactionnel est agité pendant 2 heures à +5°C puis lavé par 10 cm³ d'eau, séché sur sulfate de magnésium, filtré et concentré à sec sous pression réduite (2,7 kPa). Le résidu obtenu est chromatographié sur une colonne de gel de silice (0,04 mm-0,06 mm, diamètre 2 cm, hauteur 35 cm), en éluant par de l'acétate d'éthyle et en recueillant des fractions de 30 cm³. Les fractions 8 à 27 sont réunies, concentrées à sec sous pression réduite (2,7 kPa). Le résidu est cristallisé dans un mélange de 4 cm³d'acétonitrile et de 20 cm³ d'éther éthylique. Les cristaux sont essorés, séchés sous pression réduite (2,7 kPa). On obtient 0,70 g de [(diméthylamino-2 phényl)acetyl]-2 diphényl-7,7 perhydroisoindolol-4-(3aR,4S,7aR), sous la forme d'un solide blanc fondant à 160°C, [α]⁰D= -162° (c = 0,5, méthanol).

### EXEMPLE 10

En opérant selon le mode opératoire de l'exemple 9 à partir de 0,26 g d'acide diméthylamino-2 phénylacétique et de 0,50 g de chlorhydrate de diphényl-7,7 perhydroisoindolol-4-(3aR,4R,7aR), on obtient 0,21 g de [(diméthylamino-2 phényl)acétyl]-2 di-phényl-7,7 perhydroisoindolol-4-(3aR,4R,7aR), sous la forme d'un solide blanc fondant à 204°C, [α]⁰D = -212° (c = 0,5, méthanol).

Le chlorhydrate de diphényl-7,7 perhydroisoindolol-4-(3aR,4R,7aR) peut être préparé par hydrogénation d'une suspension de 0,70 g de benzyl-2 diphényl-7,7 perhydroisoindolol-4- (3aR,4R,7aR) dans 30 cm³ de méthanol et 2,0 cm³ d'acide chlorhydrique 1N à pression atmosphérique pendant 20 heures à 20°C en présence de 0,12 g d'hydroxyde de palladium à 20% sur noir de carbone. Le mélange réactionnel est filtré, concentré à sec sous pression réduite (2,7 kPa), l'huile obtenue est concrètée avec de l'éther éthylique. La suspension est filtrée, le solide essoré et séché sous pression réduite (2,7 kPa). On obtient 0,52 g de chlorhydrate de diphényl-7,7 perhydroisoindolol-4-(3aR,4R,7aR), sous la forme d'un solide blanc fondant à 220°C (avec décomposition).

Spectre infra-rouge (bandes caractéristiques, cm⁻¹): 3400, 3090, 3050, 3025, 3000-2800, 1600, 1580, 1495, 1465, 985, 750, 700.

Spectre RMN du proton (DMSO-d₆, signaux principaux): 1,06 (t large, J=14, 1H, H en 5); 1,66 (d large, J=14, 1H, H en 5); 2,17 (d large, J=14, 1H, CH₂ en 6); 3,8 (s large, 1H, H en 4); 5,3 (mf, 1H, OH); 7,05 à 7,45 (mt, 10H, aromatiques); 8,4 et 9,43 (mf, 2H, NH₂ ⁺).

Le benzyl-2 diphényl-7,7 perhydroisoindolol-4-(3aR,4R,7aR) peut être préparé de la façon suivante:

A une solution refroidie à 0°C de 1,3 g de benzyl-2 diphényl-7,7 perhydroisoindolone-4-(3aR,7aR) dans 6,0 cm³de tetrahydrofuranne, on ajoute en 5 minutes 4,0 cm³ d'une solution 1M de tri-sec-butyl borohydrure de lithium dans le tetrahydrofuranne. Le mélange réactionnel après agitation 3 heures à 0°C est à nouveau additionné de 0,5 cm³ de la solution 1M de borohydrure. Après 1 heure à 0°C, puis ajout de 50 cm³ d'eau et 50 cm³ d'acétate d'éthyle, la phase organique est décantée, lavée par 20 cm³ d'eau, séchée sur sulfate de magnésium et concentrée à sec sous pression réduite (2,7 kPa). L'huile obtenue est cristallisée dans 30 cm³ d'oxyde de diisopropyle, les cristaux sont essorés, séchés sous pression réduite (2,7 kPa). On obtient 0,70 g de benzyl-2 diphényl-7,7 perhydroisoindolol-4-(3aR,4R,7aR), sous la forme de cristaux blancs fondant à 154°C.

Le benzyl-2 diphényl-7,7 perhydroisoindolone-4-(3aR,7aR) peut être préparé de la façon suivante:

A une solution refroidie à 0°C de 21,7 g de chlorhydrate de diphényl-7,7 perhydroisoindolone-4-(3aR,7aR), dans 300 cm³ de di-chlorométhane, et 18,5 cm³ de triéthylamine, on ajoute 7,9 cm³ de bromure de benzyle. Le mélange réactionnel après agitation 1 heure à 0°C et 2 heures à 20°C est lavé par 50 cm³ d'eau, séché sur sulfate de magnésium, concentré à sec sous pression réduite (2,7 kPa). Le résidu est chromatographié sur une colonne de gel de silice (0,04-0,06 nm,diamètre 5 cm, hauteur 40 cm) en éluant sous une pression de 0,6 bar d'azote, par un mélange d'acétate d'éthyle et de cyclohexane (75/25 en volumes) et en recueillant des fractions de 250 cm³. Les fractions 3 à 6 sont réunies et concentrées à sec sous pression réduite (2,7 kPa). On obtient 22,1 g de benzyl-7,7 perhydroisoindolone-4-(3aR,7aR), sous la forme d'un solide blanc fondant à 124°C. [α]⁰D = -279°.

### EXEMPLE 11

A une solution de 0,86 g de bromhydrate d'acide (pyrrolidino-2)-phényl-acétique dans 20 cm³ de dichlorométhane sec, refroidie à + 4°C; on ajoute 0,42 cm³ de triéthylamine et 0,49 g de carbonyldiimidazole. On agite une heure à + 4°C, puis on ajoute une solution de 1 g de chlorhydrate de diphényl-7,7 perhydroisoindolol-4-(3aR,4S,7aR) et 0,42 cm³ de triéthylamine dans 10 cm³ de dichlorométhane sec. Le mélange réactionnel est agité à température ambiante pendant 24 heures, puis puis lavé deux fois par 10 cm³ d'eau, puis par une solution aqueuse d'hydrogénocarbonate de sodium, séché sur sulfate de magnésium, filtré et concentré à sec sous pression réduite (2,7 kPa). Le produit, obtenu sous forme de base, est converti en chlorhydrate par dissolution dans la quantité minimale d'acétone et traitement par une solution d'acide chlorhydrique dans l'éther éthylique et ajout d'éther éthylique. Le solide obtenu est trituré dans l'éther éthylique, puis séché. On obtient 0,2 g de chlorhydrate de diphényl-7,7[(pyrrolidino-2 phényl) acétyl]-2 perhydroisoindolol-4-(3aR,4S,7aR) sous forme de solide beige.

Spectre infra-rouge (KBr, bandes caractéristiques, cm⁻¹): 3085, 3050, 3025, 2945, 2880, 2750, 2250, 1640, 1600, 1495, 1445, 1060, 755, 730, 700.

Spectre RMN du proton (DMSO-d₆): 0,92 et 1,72 (2 mt, 2x1H, CH₂- en 5) ; 2,17 (mt, 4H, 2 CH₂ en 3 et 4 du pyrrolidino) ; 7 à 7,8 (mt, 14H, aromatiques).

### _{EXEMPLE 12}

En opérant comme décrit précédemment dans l'exemple 9, à partir de 1,82 g d'acide (méthoxy-2 phényl)acétique et de 3,29 g de chlorhydrate de diphényl-7,7 perhydroisoindolol-4-(3aR,4S,7aR), on obtient 3,9 g de [(méthoxy-2 phényl)acétyl)-2 diphényl-7,7 perhydroisoindolol-4-(3aR,4S,7aR) sous la forme d'un solide blanc, fondant à 246°C. [α]⁰D = -174° (c = 0,37; méthanol)

### _{EXEMPLE 13}

A une solution de 0,41 g d'acide (méthoxy-2 phényl)-2 propionique-(S) dans 15 cm³ de dichlorométhane sec, refroidie à + 4°C, on ajoute 0,37 g de carbonyldiimidazole. On agite une heure à + 4°C, puis on ajoute une solution de 0,75 g de chlorhydrate de diphényl-7,7 perhydroisoindolol-4-(3aR,4S,7aR), Le mélange réactionnel est agité à température ambiante pendant 20 heures, puis puis lavé deux fois par 10 cm³ d'eau, séché sur sulfate de magnésium, filtré et concentré à sec sous pression réduite (2,7 kPa). Le résidu est chromatographié sur colonne de gel de silice (granulométrie 0,04-0,06 mm diamètre 3,6 cm, hauteur 37 cm), en éluant sous une pression de 0,5 bar d'azote par un mélange d'acétate d'éthyle et de cyclohexane (50/50 en volumes) et en recueillant des fractions de 50 cm³. Les fractions 21 à 41 sont réunies et concentrées à sec sous pression réduite (2,7 kPa). Le résidu est trituré dans de l'oxyde d'isopropyle puis séché. On obtient 0,3 g de diphényl-7,7 [(méthoxy-2 phényl)-2 propionyl-(S)]-2 perhydroisoindolol-4-(3aR,4S,7aR) sous la forme d'une meringue blanche.

Spectre infra-rouge (KBr, bandes caractéristiques, cm⁻¹): 3090, 3060, 3030, 2940, 2875, 2840, 1630, 1600, 1495, 1445, 1245, 1060, 755, 730, 700.

Spectre RMN du proton (DMSO-d₆) (à température ambiante, on observe le mélange des deux rotamères): 0,9-1,8 (mt, 2H, CH₂ en 5); 1,14 et 1,23 (2d, J=7, 3H, CH₃); 3,55 et 3,65 (2s, 3H, OCH₃); 3,85 et 4,23 (2mt, 1H, -COCHCH₃-); 6,8 à 7,5 (mt, 14H, aromatiques).

Le chlorhydrate de diphényl-7,7 perhydroisoindolol-4-(3aR,4S,7aR) peut être obtenu de la manière suivante :

A une solution de 2 g de diphényl-7,7 tert-butyloxycarbonyl-2 perhydroisoindolol-4-(3aR,4S,7aR) dans 20 cm³ de dioxanne on ajoute une solution de 40 cm³ de dioxanne chlorhydrique 6,3 N et on agite à température ambiante pendant 5 heures. Le mélange réactionnel est concentré à sec sous pression réduite (2,7 kPa), trituré dans l'acétonitrile, filtré et séché. On obtient 1,57 g de chlorhydrate de diphényl-7,7 perhydroisoindolol-4-(3aR,4S,7aR) sous forme de cristaux blancs fondant à 266°C.

Le diphényl-7,7 tert-butyloxycarbonyl-2 perhydroisoindolol-4-(3aR,4S, 7aR) et le diphényl-7,7 tert-butyloxycarbonyl-2 perhydro-isoindolol-4-(3aR,4R,7aR) peuvent être obtenus de la manière suivante :

A une solution refroidie à + 4°C de 17,8 g de diphényl-7,7 tert-butyloxycarbonyl-2 perhydroisoindolone-4-(3aR,7aR) dans un litre de méthanol on ajoute goutte à goutte pendant 40 minutes une solution de 1 g de borohydrure de sodium dans 200 cm³ de méthanol puis 10 gouttes de lessive de soude. Le mélange réactionnel est agité 3 heures à + 4°C, puis on ajoute 2 cm³ d'une solution aqueuse d'acide chlorhydrique 0,1 N et on concentre à sec sous pression réduite (2,7 kPa). Le résidu est dissous dans 350 cm³ de dichlorométhane, lavé par 100 cm³ d'eau, puis par 50 cm³de solution saturée de chlorure de sodium, séché sur sulfate de magnésium, concentré à sec sous pression réduite (2,7 kPa). Le résidu est cristallisé dans 40 cm³ d'éther éthylique. Les cristaux obtenus sont essorés et séchés. On obtient 8,4 g de diphényl-7,7 tert-butyloxycarbonyl-2 perhydroisoindolol-4-(3aR,4S,7aR) sous la forme de cristaux blancs fondant à 190°C. Les eaux mères de cristallisation sont concentrées à sec sous pression réduite (2,7 kPa). Le résidu est chromatographié sur colonne de gel de silice (granulométrie 0,04-0,06 mm, diamètre 4 cm, hauteur 33 cm), en éluant sous une pression de 0,4 bar d'azote par un mélange de dichlorométhane et de méthanol(96/4 en volumes) et en recueillant des fractions de 20 cm³. Les fractions 18 à 21 sont réunies et concentrées à sec sous pression réduite (2,7 kPa). On obtient 1,88 g de diphényl-7,7 tert-butyloxycarbonyl-2 perhydroisoindolol-4-(3aR,4R,7aR) sous forme de meringue blanche. Les fractions 26 à 31 sont réunies et concentrées à sec sous pression réduite (2,7 kPa). Le résidu est cristallisé dans 5cm³ d'éther éthylique. On obtient 2,88 g supplémentaires de diphényl-7,7 tert-butyloxycarbonyl-2 perhydroisoindolol-4-(3aR,4S,7aR) sous forme de cristaux blancs fondant à 190°C.

Le diphényl-7,7 tert-butyloxycarbonyl-2 perhydroisoindolone-4-(3aR,7aR) peut être obtenu de la manière suivante :

A une solution de 20 g de chlorhydrate de diphényl-7,7 perhydroisoindolone-4-(3aR,7aR) dans 100 cm³ de dichlorométhane sec et 6,17 cm³ de triéthylamine on ajoute successivement 0,74 g de diméthylamino-4 pyridine, puis 14,7 g de di-tert-butyl dicarbonate. Le mélange réactionnel est agité pendant 24 heures à température ambiante, puis lavé par une solution aqueuse d'acide citrique,puis par une solution aqueuse d'hydrogénocarbonate de sodium, séché sur sulfate de magnésium, filtré et concentré à sec sous pression réduite (2,7 kPa). Le résidu est cristallisé dans 90 cm³ d'éther éthylique. Les cristaux sont essorés, lavés par 10 cm³ d'éther éthylique puis séchés. On obtient 14,1 g de diphényl-7,7 tert-butyloxycarbonyl-2 perhydroisoindolone-4-(3aR,7aR), sous la forme de cristaux blancs fondant à 119°C.

### EXEMPLE 14

A une solution refroidie à + 10°C de 1 g de diphényl-7,7 perhydroisoindolol-4-(3aR,4S,7aR), 0,97 g d'acide (diméthylamino-3 propoxy)-2 phénylacétique, 0,05 g d'hydroxy-1 benzotriazole dans 50 cm³ de dichlorométhane on ajoute 0,766 g de (diméthylamino-3 propyl)-1 éthyl-3 carbodiimide. Le mélange réactionnel est agité 90 minutes 20°C puis lavé 2 fois par 50 cm³ d'eau et par 50 cm³ de solution saturée de chlorure de sodium. La phase organique est séchée sur sulfate de magnésium et concentrée à sec sous pression réduite (2,7 kPa). Le résidu est chromatographié sur une colonne de gel de silice (granulométrie 0,2-0,063 mm, diamètre 2,9 cm, hauteur 23 cm) en éluant sous une pression de 0,7 bar d'azote par des mélanges de dichloro-1,2 éthane et de méthanol (1 litre à 90/10 en volumes et 1,5 litre à 70/30 en volume) et en recueillant des fractions de 25 cm³. Les fractions 10 à 84 sont réunies et concentrées à sec sous pression réduite (2,7 kPa) pour donner 1,1 g de {[(diméthylamino-3 propoxy)-2 phényl]acétyl}-2 diphényl-7,7 perhydroisoindolol-4-(3aR,4s,7aR) sous la forme d'une meringue crème.

Spectre infra-rouge (KBr, bandes caractéristiques, cm⁻¹): 3080, 3050, 3020, 2940, 2870., 2815, 2765, 1635, 1600, 1490, 1455, 1445, 1245, 1065, 750, 730, 700.

Spectre RMN du proton (DMSO-d₆) à 433°K : 1,06 et 1,76 (2mt, 2x1H, CH₂ en 5) ; 2,27 (s, 6H, N(CH₃)₂) ; 3,9 (d, J=11, 1H, 1H du CH₂ en 3) ; 6,8 à 7,5 (mt, 14H, aromatiques)

Une solution de 100 g d'acide hydroxy-2 phénylacétique, 75 cm³ d'alcool benzylique et 0,5 g d'acide paratoluène sulfonique dans 1400 cm³ de toluène est chauffée au reflux pendant 2 heures en éliminant l'eau formée. Après refroidissement, traitement par 3 g de noir animal et filtration, le mélange réactionnel est concentré à 150 cm³ et additionné de 300 cm³ d'oxyde d'isopropyle. Les cristaux obtenus par refroidissement à 0°C sont essorés lavés et séchés pour donner 82,5 g d'hydroxy-2 phénylacétate de benzyle. A une solution 153 g de cet ester dans un mélange de 500 cm³ de dibromo-1,3 propane et 2500 cm³ d'acétonitrile on ajoute 174 g de carbonate de potassium et l'on chauffe le mélange 17 heures au reflux. Le mélange réactionnel est refroidi, filtré et concentré à sec sous pression réduite (2,7 kPa). Le résidu est repris par 500 cm³ d'acétate d'éthyle et la phase organique est lavée par 2 fois par 400 cm³ d'eau et 2 fois par 250 cm³ de solution saturée de chlorure de sodium puis séchée et concentrée à sec sous pression réduite (2,7 kPa). Le résidu est chromatographié sur une colonne de gel de silice (granulométrie 0,2-0,063 mm, diamètre 9 cm, hauteur 55 cm) en éluant par un mélange de cyclohexane et d'acétate d'éthyle (95/5 en volumes) et en recueillant des fractions de 500 cm³. Les fractions 12 à 18 sont réunies et concentrées à sec sous pression réduite (2,7 kPa) pour donner 90 g de (bromo-3 propoxy)-2 phénylacétate de benzyle sous la forme d'une huile jaune. Une solution de 40 g de ce produit dans 500 cm³ d'acétonitrile est chauffée en autoclave avec 27 g d'iodure de sodium et 90 g de diméthylamine pendant 16 heures à 80°C. Le mélange réactionnel est refroidi, filtré et concentré à sec sous pression réduite (2,7 kPa). Le résidu est purifié par passage acide-base pour donner 29,3 g de (diméthylamino-3 propoxy)-2 phénylacétate de benzyle sous la forme d'une huile jaune. L'hydrogènation de cet ester à pression ordinaire à 40°C dans l'acétate d'éthyle en présence d'hydroxyde de palladium puis cristallisation dans l'acétate d'éthyle conduisent à 17,5 g d'acide (diméthylamino-3 propoxy)-2 phénylacétique sous la forme de cristaux blanc fondant à 98°C.

### EXEMPLE 15

En opérant comme dans l'exemple 2, mais à partir de chlorhydrate de l'acide [(isopropylamino-3 propoxy)-2 phényl]acétique et de chlorhydrate de diphényl-4,4 fluoro-7 perhydroisoindole-(3aR,7R,7aR) on obtient une huile jaune après purification par chromatographie sur colonne de gel de silice Merck (granulométrie 0,04-0,06 mm, diamètre 3 cm, hauteur 25 cm), en éluant sous une pression de 0,5 bar d'azote par un mélange de dichlorométhane et de méthanol (90/10 en volumes) et en recueillant des fractions de 20 cm³. Le chlorhydrate de cette huile conduit à 1'[[[(isopropylamino-1)-3 propoxy-2] phényl] acétyl]-2 diphényl-4,4 fluoro-7 perhydroisoindole-(3aR,7R,7aR), chlorhydrate sous forme d'un solide marron.

Spectre infra-rouge (KBr, bandes caractéristiques, cm⁻¹) : 3090+3060+3030, 2950+2885, 2860-2250, 1640, 1605+1495, 1455+1445, 1245, 750+700.

Spectre RMN du proton (250 MHz, DMSO, δ en ppm et J en Hz) : (A température ambiante, on observe le mélange des deux rotamères); 1 à 1,35 (mt, 1H, H axial du CH₂ en 6); 1,18 et 1,25 (2d, J=7, 6H, CH₃ isopropyl); 1,8 à 2,1 (mt, H du CH₂ en 6 et CH₂ en a); 2,27 (d large, J=13,5 1H, H équatorial du CH₂ en 5); 2,4 à 4 (mt, H du CH₂ en 5, CH₂ en 3, CH₂ en 1, CH en 3a, CH en 7a, N-CH); 3,02 (mt, 2H, NCH₂ en b); 3,3 à 3,54 (2s, 2H, NCOCH₂Ar); 3,9 à 4,15 (mt, 2H, OCH₂); 4,76 et 4,8 (2d large, J=50, 1H , CHF); 6,7 à 7, 5 (mt, 14H, H aromatiques).

### EXEMPLE 16

En opérant comme dans l'exemple 2, mais à partir de chlorhydrate de l'acide [(benzylamino-3 propoxy)-2 phényl]acétique et de chlorhydrate de diphényl-4,4 fluoro-7 perhydroisoindole-(3aR,7R,7aR) on obtient une huile jaune après purification par chromatographie sur colonne de gel de silice Merck (granulométrie 0,04-0,06 mm, diamètre 3 cm, hauteur 25 cm), en éluant sous une pression de 0,5 bar d'azote par un mélange d'acétate d'éthyle et de méthanol (95/5 en volumes) et en recueillant des fractions de 20 cm³. L'oxalate acide de cette huile conduit au [[[(benzylamino-1)-3 propoxy-2] phényl] acétyl]-2 diphényl-4,4 fluoro-7 perhydroisoindole-(3aR, 7R, 7aR), oxalate acide sous forme d'un solide marron.

Spectre infra-rouge (KBr, bandes caractéristiques, cm⁻¹) : 3090+3060+3030, 2955+2885, 3250-2250, 1780+1720, 1640, 1625, 1605+1498, 1455+1445, 1245, 750+700.

Spectre RMN du proton (250 MHz, DMSO, δ en ppm et J en Hz) : (A température ambiante, on observe le mélange des deux rotamères); 1 à 1,4 (mt, 1H, H axial du CH₂ en 6); 1,8 à 4,2 (mt, autre H du CH₂ en 6, CH₂ en 5, CH₂ en 3, CH₂ en 1, CH en 3a et CH en 7a, OCH₂-CH₂-CH₂N); 3,27 et 3,53 (2s, 2H, NCOCH₂Ar); 4,1 et 4,18 (2s, 2H, NCH₂Ar), 4,78 et 4,81 (2dmt, J=50, 1H, CH-F); 6,7 à 7,6 (mt, 19H, aromatiques).

### _{EXEMPLE 17}

En opérant comme dans l'exemple 2, mais à partir de 1,26 g de chlorhydrate de l'acide [(diéthylamino-3 propoxy)-2 phényl]acétique et de 1,2 g de chlorhydrate de diphényl-4,4 fluoro-7 perhydroisoindole-(3aR,7R,7aR) on obtient après purification par chromatographie sur une colonne de gel de silice, 1,5 g de chlorhydrate de {[(diéthylamino-3 propoxy)-2 phényl]acétyl)-2 diphényl-4,4 fluoro-7 perhydroisoindole-(3aR,7R,7aR), sous la forme d'un solide blanc.

Spectre infra-rouge (KBr, bandes caractéristiques, cm⁻¹) : 3090, 3060, 3030, 2950, 2885, 2800, 2200, 1640, 1600, 1498, 1455, 1250, 1050, 750, 700.

Spectre RMN du proton (250 MHz, DMSO d6 + CD₃COOD, à température ambiante on observe le mélange de deux rotamères): 1 à 1,35 (mt, 1H, H-6 axial); 1,18 et 1,23 (2t, J= 6,5, 6H en totalité, -CH₂CH₃); 1,8 à 2,2 (mt, 3H, H-6' et CH₂-CH₂-CH₂); 2,26 (d large, J= 13,5, 1H, H équatorial en 5); 2,4 à 4 (mt, 6H, CH₂ en 1, CH₂ en 3, H-3a et H-7a); 2,8 (td, J= 13,5 et 3, 1H, H-5 axial); 3 à 3,2 (mt, 6H, CH₂-N(CH₂CH₃)₂); 3,3 et 3,53 (2s, 2H, =NCOCH₂Ar); 3,9 à 4,1 (mt, 2H, OCH₂); 4,76 et 4,81 (2d larges, J= 50, 1H en totalité, =CHF); 6,7 à 7,5 (mt, 14H, aromatiques).

Le chlorhydrate de l'acide [(diéthylamino-3 propoxy)-2 phényl]acétique peut être préparé en opérant comme il est décrit dans l'exemple 20 à partir de 1,24 g de [(diéthylamino-3 propoxy)-2 phényl]acétate de méthyle et de 20 cm³ d'acide chlorhydrique 6N. On obtient 1,26 g de chlorhydrate de l'acide [(diéthylamino-3 propoxy)-2 phényl]acétique, sous la forme d'un solide marron clair.

Spectre infra-rouge(KBr, bandes caractéristiques, cm⁻¹) : 3250-2250, 3100-3000, 3000-2850, 1722, 1600, 1585, 1495, 1472, 1450, 1385, 1250, 760.

Spectre RMN du proton (250 MHz, DMSO-d₆) : 1,25 (t, J= 7,5, 6H, 2xCH₂CH₃) ; 2,14 (mt, 2H, CH₂-CH₂-CH₂) ; 3,05 à 3,3 (mt, 6H, CH₂-N(CH₂CH₃)₂) ; 3,53 (2s, 2H, CH₂COO) ; 4,08 (t, J=6, 2H, OCH₂) ; 6,33 (mt, 2H, aromatiques en 3 et 5) ; 7,23 (mt, 2H, aromatiques en 4 et 6); 10,62 (mf, 1H, -NH⁺<); 12,2 (mf, 1H, COOH).

Le [(diéthylamino-3 propoxy)-2 phényl]acétate de méthyle peut être préparé comme il est décrit dans l'exemple 20, à partir de 1,8 g de (bromo-3 propoxy)-2 phényl acétate de méthyle et de 0,66 cm³ de diéthylamine. On obtient après purification par chromatographie sur une colonne de gel de silice, 1,24 g de [(diéthylamino-3 propoxy)-2 phényl]acétate de méthyle, sous la forme d'une huile orange qui cristallise à température ordinaire.

Spectre infra-rouge (CCl₄, bandes caractéristiques, cm⁻¹) : 3070, 3030, 2970, 2930, 2875, 2800, 1740, 1605, 1590, 1495, 1475, 1455, 1435, 1250.

Spectre RMN du proton (250 MHz, CDCl₃) : 1,10 (t, J= 7,5, 6H, 2xCH₂CH₃) ; 1,96 (mt, 2H, CH₂-CH₂-CH₂) ; 2,62 (q, J=7, 4H, N(CH₂CH₃)₂) ; 2,68 (t, J=7, 2H, CH₂-NEt₂) ; 3,64 (2s, 2H, CH₂COO) ; 3,7 (s, 3H, COOCH₃) ; 4,03 (t, J=6, 2H, OCH₂) ; 6,9 (mt, 2H, aromatiques en 3 et 5), 7,22 (mt, 2H, aromatiques en 4 et 6).

### EXEMPLE 18

En opérant comme dans l'exemple 2, mais à partir de 1,37 g de l'acide {[N-(hydroxy-2 éthyl) N-méthylamino]-3 propoxy-2}phénylacétique et de 1,2 g de chlorhydrate de diphényl-4,4 fluoro-7 perhydroisoindole-(3aR,7R,7aR) on obtient après purification par chromatographie sur une colonne de gel de silice, 0,86 g de {{[N-(hydroxy-2 éthyl) N-méthylamino]-3 propoxy-2}phénylacétyl}-2 diphényl-4,4 fluoro-7 perhydroisoindole-(3aR,7R,7aR), sous la forme d'une meringue blanche.

Spectre infra-rouge(KBr, bandes caractéristiques, cm⁻¹) : 3425, 3105, 3090, 3060, 3030, 2950, 2885, 2850, 2800, 1640, 1600, 1495, 1455, 1445, 1250, 1050, 755, 700.

Spectre RMN du proton (400 MHz, DMSO-d₆ + CD₃COOD d4, à 303°K) : (A cette température on observe un mélange de rotamères); 1,14 (dt large, J=46 et 13,5, 1H, H-6 axial) ; 1,85 (mt, 1H, H-6 équatorial) ; 2,03 (mt, 2H, CH₂-CH₂-CH₂) ; 2,20 (mt, 1H, H-5) ; 2,3 à 3,8 (mt, H-5', CH₂-1 et CH₂-3, H-7a et H-3a, >NCOCH₂Ar, -CH₂-N-CH₂CH₂O-) ; 2,75 et 2,80 (2s, 3H, >N-CH₃) ; 3,8 à 4 (mt, 2H, ArOCH₂) ; 4,7 et 4,74 (2 dmt, J=50, 1H, CHF) ; 6,7 à 7,4 (mt, 14H, aromatiques).

L'acide {[N-(hydroxy-2 éthyl) N-méthylamino]-3 propoxy-2} phénylacétique peut être préparé en opérant comme décrit dans l'exemple 20 à partir de 2,1 g de {{[N-(hydroxy-2 éthyl) N-méthylamino]-3 propoxy-2}phényl}acétate de méthyle et de 30 cm³ d'acide chlorhydrique 6N. On obtient 2,27 g d'acide {[N-(hydroxy-2 éthyl) N-méthylamino]-3 propoxy-2}phénylacétique, sous la forme d'une huile rouge.

Spectre infra-rouge (entre lamelles, bandes caractéristiques, cm⁻¹): 1725, 1605, 1590, 1498, 1475, 1455, 1465, 1250, 1055, 760.

Spectre RMN du proton (400 MHz, DMSO-d₆, 383°K): 1,98 (qt, J=6,5, 2H, CH₂-CH₂-CH₂) ; 2,84 (s, 3H, >N-CH₃) ; 3,15 à 3,4 (mt, 4H, NCH2) ; 3,56 (s, 2H, CH₂COO) ; 3,84 (t, J=6, CH₂OH) ; 4,1 (t, J=6,5, ArOCH₂); 6,96 (mt, 2H, aromatiques en 3 et 5) ; 7,24 (mt, 2H, aromatiques en 4 et 6).

Le {{[N-(hydroxy-2 éthyl) N-méthylamino]-3 propoxy-2}phényl}acétate de méthyle peut être préparé comme il est décrit dans l'exemple 20, à partir de 1,8 g de (bromo-3 propoxy)-2 phényl acétate de méthyle et de 0,5 cm³ de (méthylamino)-2 éthanol. On obtient après purification par chromatographie sur une colonne de gel de silice, 0,5 g de {[N-(hydroxy-2 éthyl) N-méthylamino]-3 propoxy-2}phényl acétate de méthyle, sous la forme d'une huile rosâtre.

Spectre infra-rouge (CH₂Cl₂, bandes caractéristiques, cm⁻¹) : 3460, 3050, 3030, 3000-2850, 2810, 1738, 1600, 1590, 1498, 1455, 1435, 1250.

Spectre RMN du proton (250 MHz, CDCl₃) : 2,07 (mt, 2H, CH₂-CH₂-CH₂) ; 2,2 à 2,8 (mf, 1H, OH) ; 2,43 (s, 3H, >N-CH₃); 2,72 (t, J=5,5, 2H, NCH₂CH2OH) ; 2,76 (t, J=7, 2H, CH₂N) ; 3,64 (s, 2H, CH₂COO) ; 3,71 (s, 3H, CO₂CH₃) ; 3,71 (t, J=5,5, CH₂OH) ; 4,04 (t, J=6, ArOCH₂) ; 6,32 (mt, 2H, aromatiques en 3 et 5) ; 7,24 (mt, 2H, aromatiques en 4 et 6).

### EXEMPLE 19

En opérant comme dans l'exemple 2, mais à partir de 1,5 g de chlorhydrate de l'acide [(diisopropylamino-3 propoxy)-2 phényl] acétique et de 1,3 g de chlorhydrate de diphényl-4,4 fluoro-7 perhydroisoindole-(3aR,7R,7aR) on obtient après purification par chromatographie sur colonne de gel de silice 1,57 g de chlorhydrate de {[(diisopropylamino-3 propoxy)-2 phényllacéty}-2 diphényl-4,4 fluoro-7 perhydroisoindole-(3aR,7R,7aR), sous la forme d'un solide blanc.

Spectre infra-rouge(KBr, bandes caractéristiques, cm⁻¹) : 3090, 3060, 3030, 2950, 2855, 2800-2200, 1640, 1605, 1495, 1455, 1445, 1247, 755, 700.

Spectre RMN du proton (250 MHz, DMSO-d₆ + CD₃COOD, à température ambiante on observe le mélange de deux rotamères) : 1 à 1,4 (mt, 1H, H-6 axial) ; 1,2 à 1,4 (mt, 12H, -CH₃ isopropyles); 1,8 à 2,2 (mt, 3H, H-6' et CH₂-CH₂-CH₂) ; 2,25 (d large, J=13,5, 1H, H-5 équatorial) ; 2,4 à 4 (mt, H-5', CH₂-3, CH₂-1, H-3a, H-7a et -N(CH(CH₃₎₂)₂) ; 3,15 (mt, 2H, -CH₂N<) ; 3,3 et 3,55 (2s, 2H, >NCOCH₂Ar) ; 3,9 à 4,1 (mt, 2H, OCH₂) ; 4,75 et 4,79 (2d, J=50, 1H, CHF); 6,7 à 7,5 (mt, 13H, aromatiques).

Le chlorhydrate de l'acide [(diisopropylamino-3 propoxy)-2 phényl] acétique peut être préparé en opérant comme il est décrit dans l'exemple 20 à partir de 1,50 g de [(diisopropylamino-3 propoxy)-2 phényl] acétate de méthyle et de 30 cm³ d'acide chlohydrique 6N. On obtient 1,5g de chlorhydrate de l'acide [(diisopropylamino-3 propoxy)-2 phényl] acétique, sous la forme d'une huile marron.

Spectre infra-rouge (KBr, bandes caractéristiques, cm⁻¹) : 3500-2250, 3100-3000, 3000-2850, 1727, 1605, 1595, 1500, 1470, 1455, 1425, 1250, 760.

Spectre RMN du proton (250 MHz, DMSO-d₆) : 1,31 et 1,36 (2d, J=6,5, 12H, -CH₃ isopropyles); 2,2 (mt, 2H, CH₂-CH₂-CH₂) ; 3,21 (mt, J=7, 2H, -CH₂N<) ; 3,53 (s, 2H, >NCOCH₂Ar) ; 3,61 (mt, 2H, -N(CH(CH₃)₂)₂) ; 4,07 (t, J=6, 2H, OCH₂) ; 6,91 (mt, 2H, aromatiques en 3 et 5) ; 7,22 (mt, 2H, aromatiques en 4 et 6); 9,98 (mf, 1H, -NH⁺<).

Le [(diisopropylamino-3 propoxy)-2 phényl] acétate de méthyle peut être préparé comme il est décrit dans l'exemple 20, à partir de 2,65 g de (bromo-3 propoxy)-2 phényl acétate de méthyle et de 1,98 cm³de diisopropylamine. On obtient 1,31 g de [(diisopropylamino-3 propoxy)-2 phényl] acétate de méthyle, sous la forme d'une huile jaune.

Spectre infra-rouge (CCl₄, bandes caractéristiques, cm⁻¹) : 3070, 3050, 3030, 2970, 2930, 2875, 1743, 1605, 1590, 1495, 1470, 1455, 1435, 1385, 1360, 1250.

Spectre RMN du proton (250 MHz, DMSO-d₆): 0,97 (d, J=7, 12H, -CH₃ isopropyles); 1,72 (quintuplet, J=7, 2H, CH₂-CH₂-CH₂) ; 2,55 (t, J=7, 2H, -CH₂N<) ; 2,97 (mt, 2H, -N(CHMe)₂) ; 3,59 et 3,62 (2s, 5H, >NCOCH₂Ar et CO₂CH₃) ; 3,97 (t, J-7, 2H, OCH₂) ; 6,32 (mt, 2H, aromatiques en 3 et 5) ; 7,22 (mt, 2H, aromatiques en 4 et 6).

### EXEMPLE 20

En opérant comme dans l'exemple 2, mais à partir de 1,45 g de chlorhydrate de l'acide [(pipéridino-3 propoxy)-2 phényl] acétique et de 1,30 g de chlorhydrate de diphényl-4,4 fluoro-7 perhydroisoindole-(3aR,7R,7aR) on obtient après purification par chromatographie sur colonne de gel de silice 1,44 g de chlorhydrate de {[(pipéridino-3 propoxy)-2 phényl]acétyl}-2 diphényl-4,4 fluoro-7 perhydroisoindole-(3aR,7R,7aR), sous la forme d'un solide blanc.

Spectre infra-rouge(KBr, bandes caractéristiques, cm⁻¹) : 3090, 3060, 3030, 2955, 2885, 2800-2200, 1640, 1605, 1498, 1455, 1445, 1250, 755, 700.

Spectre RMN du proton (250 MHz, DMSO-d₆ à 433°K) : 1,27 (dt large, J= 46 et 13,5, H-6 axial); 1,5 à 2,1 (mf, 7H, N(CH₂CH₂)₂CH₂ et H-6'); 2,23 (mf, 2H, CH₂-CH₂-CH₂); 2,32 (d large, J= 13,5, 1H, H-5 équatorial) ; 2,64 (td, J= 13,5 et 3, 1H, H-5 axial); 2,7 à 4 (mt, CH₂-1, CH₂-3, H-3a, H-7a et N(CH₂CH₂)₂CH₂); 3,13 (t, J=7,5, 2H, -CH₂N<); 3,79 (AB, 2H, NCOCH₂Ar); 4,08 (mf, 2H, OCH₂); 4,82 (d large, J= 50, 1H, CHF); 6,8 à 7,5 (mt, 14H, aromatiques).

Le chlorhydrate de l'acide [(pipéridino-3 propoxy)-2 phényl] acétique peut être préparé de la façon suivante:

A 2,0 g de [(pipéridino-3 propoxy)-2 phényl] acétate de méthyle on ajoute une solution d'acide chlorhydrique 6N. Après 3 heures de reflux la solution est concentrée à sec à 50°C sous pression réduite. Le résidu est ensuite repris par 30 cm³ de toluène puis la solution est concentrée à sec. Après un nouveau traitement avec du toluène, on obtient 1,9 g de chlorhydrate de l'acide [(pipéridino-3 propoxy)-2 phényl] acétique sous la forme d'un solide blanc, utilisé tel quel à l'étape suivante.

Le [(pipéridino-3 propoxy)-2 phényl] acétate de méthyle peut être préparé de la façon suivante:

A une solution de 2,87 g de (bromo-3 propoxy)-2 phényl acétate de méthyle dans 30 cm³ d'acétonitrile, on ajoute successivement 1,51 g de carbonate de potassium, 0,3 g d'iodure de sodium, puis 1,0 cm³ de pipéridine. Le mélange réactionnel est porté au reflux pendant 2 heures puis filtré. Le filtrat est concentré à sec et le résidu obtenu est chromatographié sur colonne de gel de silice (granulométrie 0,04 -0,06 mm, diamètre 4 cm, hauteur 50 cm), en éluant sous une pression de 0,1 bar d'azote par un mélange d'acétate d'éthyle et de méthanol (80/20 en volumes) et en recueillant des fractions de 125 cm³. Les fractions 9 à 16 sont réunies et concentrées à sec. Le résidu est trituré dans de l'oxyde de diisopropyle, la suspension est filtrée, le filtrat est concentré à sec sous pression réduite (2,7 kPa). On obtient 2,0 g de [(pipéridino-3 propoxy)-2 phényl] acétate de méthyle sous la forme d'une huile jaune, utilisée telle quelle à l'étape suivante.

### EXEMPLE 21

En opérant selon le mode opératoire de l'exemple 20 à partir de 1,65 g de chlorhydrate de diphényl-4,4 fluoro-7 perhydroisoindole-(3aR,7R,7aR) et de 1,5 g d'acide {[(hydroxyméthyl-2 pyrrolidinyl-1-(S))-3 propoxy]-2 phényl} acétique dans 160 cm³ de dichlorométhane sec, on obtient 1,27 g de chlorhydrate de diphényl-4,4 fluoro-7 {{[(hydroxyméthyl-2 pyrrolidinyl-1-(S))-3 propoxy]-2 phényl}-acétyl}-2 perhydroisoindole-(3aR,7R,7aR) sous la forme d'une meringue blanche.

Spectre RMN du proton: 1,27 (dt large, J=46 et 13,5; 1H, H axial du -CH₂- en 6); 1,8 à 2,4 (mt, l'autre H du -CH₂- en 6 et -CH₂- de la pyrrolidine); 2,3 (d large, J=13,5; 1H, H équatorial du -CH₂- en 5); 2,66 (mt, l'autre H du -CH₂- en 5); 2,4 à 4 (mt, -CH₂- en 1, -CH₂- en 3, CH en 3a, CH en 7a et CH₂N); 3,85 (ab limite, NCOCH₂); 4 à 4,3 (mt, 4H, -CH₂-O) ; 4,84 (d large, J=50, 1H, CHF); 6,8 à 7,6 (mt, 13H aromatiques).

Spectre infra-rouge(KBr): 3420, 3105-3090-3060-3030, 2955-2885, 2800-2200, 1640, 1605-1498, 1455-1445, 1250, 1065, 1050, 755-705.

L'acide [(hydroxyméthyl-2 pyrrolidinyl-1-(S))-3 propoxy]-2 phénylacétique peut être obtenu de la manière suivante :

Une suspension de 1,65 g d'[(hydroxyméthyl-2 pyrrolidinyl-1-(S))-3 propoxy]-2 phényl acétate de méthyle dans 20 cm³ d'acide chlorhydrique 6N est portée au reflux pendant 3 heures. Le mélange réactionnel est concentré à sec sous pression réduite (2,7 kPa). Le résidu obtenu est trituré dans l'éther. On obtient 1,45 g d'acide [(hydroxyméthyl-2 pyrrolidinyl-1-(S))-3 propoxy]-2 phényl acétique sous la forme d'une huile brune.

Spectre RMN du proton: 1,7 à 2,3 (mt, 6H, -CH₂- de la pyrrolidine); 3 à 3,3 et 3,4 à 3,7 (2mt, 5H en totalité NCH₂ et NCH); 3,54 (s, 2H, CH₂COO); 3,76 (ab dédoublé, J=12,5 et 4, 2H, CH₂O); 4,06 (mt, 2H, ArOCH₂); 6,94 (mt, 2H aromatiques en ortho et para du OR); 7,22 (mt, 2H aromatiques en méta du OR); 10,35 (mf, 1H, NH⁺Cl⁻).

L'[(hydroxyméthyl-2 pyrrolidinyl-1-(S))-3 propoxy]-2 phényl acétate de méthyle peut être obtenu de la manière suivante :

A une solution de 1,8 g de (bromo-3 propoxy)-2 phényl acétate de méthyle dans 20 cm³ d'acétonitrile on ajoute successivement 0,92 g de carbonate de potassium, 0,22 g d'iodure de sodium, puis 0,63 g d'hydroxyméthyl-2 pyrrolidine-(S). Le mélange réactionnel est chauffé à 70°C pendant 5 heures, puis après refroidissement, dilué par 100 cm³ de dichlorométhane, lavé par 100 cm³ d'eau , puis par 100 cm³ d'une solution aqueuse saturée en chlorure de sodium, séché sur sulfate de magnésium et concentré à sec sous pression réduite (2,7 kPa). Le résidu est chromatographié sur colonne de gel de silice Merck (granulométrie 0,04-0,06 mm , diamètre 3 cm , hauteur 30 cm), en éluant sous une pression de 0,4 bar d'azote par un mélange d'acétate d'éthyle et de méthanol (90/10 en volumes) et en recueillant des fractions de 100 cm³. Les fractions 7 à 20 sont réunies et concentrées à sec sous pression réduite (2,7 kPa) . On obtient 1,55 g d'[(hydroxyméthyl-2 pyrrolidinyl-1-(S))-3 propoxy]-2 phényl acétate de méthyle sous la forme d'une huile.

Spectre RMN du proton : 1,5 à 2 (mt, 4H, CH₂ de la pyrrolidine); 1,84 (mt, 2H, CH₂ de la chaine propoxy); 2,2 et 3,08 (2mt, 1H chacun, NCH₂de la pyrrolidine); 2,4 et 2,35 (mt, 1H chacun, NCH₂); 2,5 (mt, 1H, NCH de la pyrrolidine); 3,22 (dd, J=11,5 et 6,5 , 1H du CH₂O); 3,44 (dd, J=11,5 et 4,5 , 1H, l'autre H du CH₂O); 3,6 (2s, 5H, CH₂COOCH₃); 4 (t, J=6, 2H, ArOCH₂); 6,92 (mt, 2H aromatiques en ortho et para du OR); 7,22 (mt, 2H aromatiques en méta du OR).

### EXEMPLE 22

En opérant comme dans l'exemple 2, mais à partir de 2,3 g de chlorhydrate de l'acide {[(delta-3-pyrrolinyl-1)-3 propoxy-2]phényl} acétique et de 2,32 g de chlorhydrate de diphényl-4,4 fluoro-7 perhydroisoindole-(3aR,7R,7aR) on obtient 3,4 g de produit brut sous la forme d'une meringue blanche. Ce produit est repris par 100 cm³ d'acétate d'éthyle, la solution obtenue est lavée par 40 cm³ d'une solution aqueuse à 5% de bicarbonate de sodium, extraite ensuite une première fois par 30 cm³ puis par 20 cm³ d'acide chlorhydrique 1N. Les extraits aqueux acides sonts réunis, alcalinisés par de la soude 4N et extraits à nouveau par deux fois 50 cm³ d'acétate d'éthyle. La phase organique est concentrée à sec sous pression réduite (2,7 kPa), le résidu est chromatographié sur une colonne de SEPHADEX LH-20 (diamètre 3 cm, hauteur 1,4 m) en éluant avec du méthanol et en recueillant des fractions de 13 cm³. Les fractions 35 à 38 sont réunies et concentrées à sec sous pression réduite (2,7 kPa). Le résidu est trituré dans de l'éther éthylique, le solide est essoré, séché sous pression réduite (2,7 kPa). On obtient 0,49 g de chlorhydrate de {{[(delta-3-pyrrolinyl-1)-3 propoxy-2]phényl} acétyl}-2 diphényl-4,4 fluoro-7 perhydroisoindole-(3aR,7R,7aR), sous la forme d'un solide blanc.

Spectre infra-rouge (KBr, bandes caractéristiques, cm⁻¹) : 3090, 3060, 3030, 2945, 2880, 2800-2200, 1640, 1600, 1495, 1455, 1445, 1245, 755, 700.

Spectre RMN du proton (200 MHz, DMSO-d₆ + CD₃COOD a 413°K): 1,32 (dt large, J=46 et 13,5, 1H, H-6 axial) ; 2,05 (mt, 1H, H-6 équatorial) ; 2,15 (qt, J=7, 2H, CH₂-CH₂-CH₂) ; 2,3 (d large, J=13,5, 1H,H-5 équatorial) ; 2,67 (td, J=13,5 et 3, 1H, H-5 axial) ; 2,6 à 3,9 (mt, CH₂-3, CH₂-1, H-3a et H-7a) ; 3,35 (t, J=7, 2H, -CH₂N<) ; 3,8 (AB limite, 2H, >NCOCH₂Ar) ; 4,07 (s, 4H, -N(CH₂CH)₂) ; 4,10 (t, J=7, 2H, CH₂O) ; 4,8 (dmt, J=50, 1H, CHF) ; 5,91 (s, 2H, CH=CH) ; 6,8 à 7,5 (mt, 14H, aromatiques).

Le chlorhydrate de l'acide {[(delta-3-pyrrolinyl-1)-3 propoxy-2]phényl}acétique peut être préparé en opérant comme il est décrit dans l'exemple 20 à partir de 8 g de {[(delta-3-pyrrolinyl-1)-3 propoxy-2]phényl}acétate de méthyle et de 100 cm³ d'acide chlorhydrique 6N. On obtient 4,6 g de chlorhydrate de (delta-3 pyrrolinyl-1)-3 propoxy-2]phényl}acétique, sous la forme d'un solide beige clair fondant à 140°C.

Le {[(delta-3-pyrrolinyl-1)-3 propoxy-2]phényl}acétate de méthyle peut être préparé comme il est décrit dans l'exemple 20 à partir de 15,6 g de (bromo-3 propoxy)-2 phényl acétate de méthyle et de 3,8 g de delta-3-pyrroline. On obtient après purification par chromatographie sur une colonne de gel de silice, 8,0 g de {[(delta-3 pyrrolinyl-1)-3 propoxy-2]phényl}acétate de méthyle, sous la forme d'une huile jaune orangée.

Spectre RMN du proton (300 MHz, CDCl₃) : 1,98 (mt, 2H, CH₂-CH₂-CH₂); 2,81 (t, J=7, 2H, -CH₂N<); 3,51 (s, 4H, -N(CH₂CH)₂); 3,64 (s, 2H, >NCOCH₂Ar); 3,7 (s, 3H, CO₂CH₃); 4,06 (t, J=6,5, 2H, CH₂O); 5,8 (s, 2H, CH=CH); 6,9 (mt, 2H, aromatiques en 3 et 5); 7,21 (mt, 2H, aromatiques en 4 et 6).

### _{EXEMPLE 23}

A une solution refroidie à 0°C de 0,4 cm³ de trifluorothio-4 morpholine dans 20 cm³ de dichlorométhane anhydre, on ajoute en 20 minutes une solution de 1,32 g de [(méthoxy-2 phényl)acétyl]-2 diphényl-7,7 perhydroisoindolol-4-(3aR,45,7aR) dans 100 cm³ de dichlorométhane anhydre. Après agitation 2 heures à 20°C le mélange réactionnel est lavé par 100 cm³ d'une solution aqueuse à 5% P/V de bicarbonate de sodium, séché sur sulfate de magnésium, concentré à sec sous pression réduite (2,7 kPa). Le résidu est chromatographié sur colonne de gel de silice (granulométrie 0,04-0,06 mm, diamètre 2,5 cm, hauteur 30 cm), en éluant sous une pression de 0,5 bar d'azote par un mélange de cyclohexane et d'acétate d'éthyle (50/50 en volumes) et en recueillant des fractions de 30 cm³. Les fractions 10 à 20 sont réunies et concentrées à sec sous pression réduite (2,7 kPa). Le résidu est lavé par de l'éther éthylique puis par de l'oxyde de diisopropyle, le solide est essoré et séché. On obtient 0,38 g de [(méthoxy-2 phényl)acétyl]-2 diphényl-4,4 fluoro-7 perhydroisoindole-(3aR,7R,7aR) sous la forme d'un solide blanc fondant à 205°C.

### _{EXEMPLE 24}

A une solution refroidie à 10°C de 0,27 cm³ de trifluorothio-4 morpholine dans 10 cm³ de dichlorométhane anhydre, on ajoute sous atmosphère d'azote en 20 minutes, une solution de 0,91 g de [(diméthylamino-2 phényl)acétyl]-2 diphényl-7,7 perhydro-isoindolol-4 mélange des isomères (3aR,4S,7aR) et (3aR,4R,7aR) (isomère 3aR,4S,7aR très majoritaire) obtenu selon l'exemple 17, dans 30 cm³ de dichlcrométhane anhydre. Après agitation 2 heures à 20°C, le mélange réactionnel est lavé successivement par 25 cm³ d'eau, 20 cm³ de sclution aqueuse d'hydroxyde de sodium 0,1N puis séché sur sulfate de magnésium, concentré à sec sous pression réduite (2,7 kPa). Le résidu est chromatographié sur colonne de gel de silice Merck (granulométrie 0,04-0,06 mm, diamètre 2 cm, hauteur 40 cm), en éluant sous une pression de 0,5 bar d'azote par un mélange de cyclohexane et d'acétate d'éthyle (70/30 puis 50/50 en volumes) et en recueillant des fractions de 20 cm³. Les fractions 22 à 25 sont réunies et concentrées à sec sous pression réduite (2,7 kPa). Le résidu est lavé par 10 cm³ d'oxyde de diisopropyle, le solide est essoré et séché. On obtient 0,12 g de [(diméthylamino-2 phényl)acétyl]-2 diphényl-4,4 fluoro-7 perhydroisoindole-(3aR,7R,7aR), sous la forme d'un solide blanc fondant à 186°C.

### EXEMPLE 25

A une suspension refroidie à 5°C de 3,0 g de [(diméthylamino-2 phényl)acétyl]-2 diphényl-7,7 perhydroisoindolone-4-(3aR,7aR) dans 150 cm³ de méthanol, on ajoute en 10 minutes une solution de 0,15 g de borohydrure de sodium et de 0,05 cm³ de solution aqueuse de soude (à 30%) dans 30 cm³ de méthanol. Après 2 heures d'agitation à 5°C, le mélange réactionnel est concentré à volume réduit (30 cm³ environ) et repris par 150 cm³ de dichlorométhane. La solution obtenue est lavée par 50 cm³ d'eau, séchée sur sulfate de magnésium, concentrée à sec à 50°C sous pression réduite (2,7 kPa). Le résidu est cristallisé dans 10 cm³ d'acétonitrile, les cristaux sont essorés, lavés avec de l'acétonitrile et séchés. On obtient 2,2 g d'un mélange de [(diméthylamino-2 phényl)acétyl]-2 diphényl-7,7 perhydroisoindolol-4-(3aR,4S,7aR) et de [(diméthyl-amino-2 phényl) acétyl]-2 diphényl-7,7 perhydroisoindolol-4- (3aR,4R,7aR), sous la forme d'un solide blanc fondant à 156°C.

### EXEMPLE 26

A une suspension refroidie à +5°C de 2,0 g de diphényl-7,7 phénylacétyl-2 perhydroisoindolone-4-(3aRS,7aRS) dans 100 cm³ de méthanol, on ajoute en 10 minutes une solution de 0,12 g de borhydrure de sodium et de 0,05 cm³ de soude 10N dans 20 cm³ de méthanol. Le mélange réactionnel est agité 2 heures à froid, puis on ajoute 0,5 cm³d'acide chlorhydrique 1N, et concentre à volume réduit, à 40°C sous pression réduite (2,7 kPA). Le résidu est repris par 50 cm³ d'eau et 150 cm³ de dichlorométhane. Après agitation la phase organique est séchée sur sulfate de magnésium, concentrée à sec. Le solide est cristallisé dans de l'acétonitrile, les cristaux sont lavés par de l'acétonitrile et de l'oxyde de diisopropyle, essorés, puis séchés. On obtient 0,24 g de diphényl-7,7 phénylacétyl-2 perhydroisoindolol-4-(3aRS,4RS,7aRS) sous la forme de cristaux blancs fondant à 220°C.

### EXEMPLE 27

Le filtrat de la cristallisation du produit obtenu à l'exemple précédent est concentré à sec. Le résidu est chromatographié sur colonne de gel de silice (granulométrie 0,04-0,06 mm, diamètre 2 cm, hauteur 35 cm), en éluant sous une pression de 0,5 bar d'azote par un mélange de dichloro-1,2 éthane et de méthanol (95/5 en volumes) et en recueillant des fractions de 50 cm³. Les fractions 14 à 17 sont réunies et concentrées à sec sous pression réduite (2,7 kPa).

On obtient 0,28 g de diphényl-7,7 phénylacétyl-2 perhydroisoindolol-4-(3aRS,4SR,7aRS) sous la forme de cristaux blancs fondant à 222°C.

### EXEMPLE 28

A une solution de 0,41 g de tétrafluoroborate de (méthoxy-2 phényl)-acétimidate d'éthyle dans 10 cm³ de dichlorométhane sec, on ajoute une solution de 0,6 g de chlorhydrate de diphényl-4,4 fluoro-7 perhydroisoindole-(3aR,7R,7aR) et de 0,51 cm³ de triéthylamine dans 10 cm³ de dichlorométhane sec. Le mélange réactionnel est chauffé 3 heures au reflux. Il est ensuite traité, après retour à tempétature ambiante, par 5 cm³ d'une solution aqueuse de carbonate de potassium à 10%; la phase organique est lavée par 10 cm³ d'eau distillée, séchée sur sulfate de magnésium et concentrée à sec sous pression réduite (2,7 kPa). Le résidu est chromatographié sur colonne de gel de silice (granulométrie 0,04-0,06 mm, diamètre 2 cm, hauteur 20 cm), en éluant sous une pression de 0,6 bar d'azote par un mélange d'acétate d'éthyle, d'acide acétique et d'eau (15/1/1 en volumes) et en recueillant des fractions de 25 cm³. Les fractions 24 à 38 sont réunies et concentrées à sec sous pression réduite (2,7 kPa). Le résidu est repris par 40 cm³ de dichlorométhane, lavé par 10 cm³ de solution aqueuse saturée de carbonate de potassium, puis par 10 cm³ de solution aqueuse saturée de chlorure de sodium. La phase organique est séchée sur sulfate de magnésium et concentrée à sec sous pression réduite (2,7 kPa). Le résidu est cristallisé dans l'oxyde d'isopropyle. Les cristaux sont essorés et séchés. On obtient 0,18 g de diphényl-4,4 fluoro-7 [imino-1 (méthoxy-2 phényl)-2 éthyl]-2 perhydroisoindole-(3aR,7R,7aR) sous la forme de cristaux blancs fondant à 184°C avec décomposition.

### EXEMPLE 29

A une solution de 2 g de diphényl-7,7 perhydroisoindolol-4-(3aR,4S,7aR) dans 30 cm³ de dichlorométhane sec, on ajoute une solution de 1,56 g de tétrafluoroborate de (méthoxy-2 phényl)-acétimidate d'éthyle et de 0,96 cm³ de triéthylamine dans 20 cm³ de dichlorométhane sec, puis on porte le mélange réactionnel au reflux pendant 2 heures. On ajoute ensuite 10 cm³ d'une solution aqueuse à 10% de carbonate de potassium, on décante, puis on lave la phase organique par 20 cm³ d'eau, on sèche sur sulfate de magnésium, on filtre et concentre à sec sous pression réduite (2,7 kPa). Le résidu est chromatographié sur colonne d'alumine (diamètre 3,6 cm, hauteur 31 cm), en éluant sous une pression de 0,1 bar d'azote par un mélange de dichlorométhane et de méthanol (95/5 en volumes) et en recueillant des fractions de 50 cm³. Les fractions 5 à 30 sont réunies et concentrées à sec sous pression réduite (2,7 kPa). Le solide obtenu est lavé à l'oxyde d'isopropyle, essoré et séché. On obtient 1,4 g de diphényl-7,7 [imino-1 (méthoxy-2 phényl)-2 éthyl]-2 perhydroisoindolol-4-(3aR,45,7aR) sous la forme de cristaux blancs, fondant à 105°C avec décomposition.

La présente invention concerne également les compositions pharmaceutiques constituées par un produit de formule générale (I) ou un sel lorsqu'ils existent, éventuellement en association avec tout autre produit pharmaceutiquement compatible, pouvant être inerte ou physiologiquement actif. Les compositions selon l'invention peuvent être utilisées par voie parentérale, orale, rectale ou topique.

Les compositions stériles pour administration parentérale qui peuvent être notamment utilisées sous forme de perfusions sont de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont des suppositoires ou des capsules rectales, qui contiennent outre le produit actif des excipients tels que le beurre de cacao, des glycérides semi-synthétiques ou des polyéthylèneglycols.

Comme compositions solides pour administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration topique peuvent être par exemple des crèmes, des pommades, ou des lotions.

En thérapeutique humaine, les produits selon l'invention peuvent être particulièrement utiles dans le traitement des douleurs d'origine traumatique, post-chirurgicale, menstruelle, céphaliques, dans les traitements de l'anxiété, des psychoses, de la maladie de Parkinson, de la schizophrénie, de la maladie d'Alzeimer, dans les traitements myorelaxants, dans les traitements des manifestations spasmodiques, douloureuses et inflammatoires des voies digestives (colites ulcéreuses, syndrome du colon irritable, maladie de Crohn), des voies urinaires (cystites) et des voies respiratoires (asthme, rhinites) ou en gynécologie et dans les traitements des migraines. Les nouveaux dérivés de l'isoindole sont également utiles dans le traitement de l'arthrite rhumatoïde et dans les troubles dus au dérèglement du système immunitaire, dans les traitements des inflammations en dermatologie tels que le psoriasis, l'herpès, les urticaires, les eczémas, les photodermatoses et dans les troubles inflammatoires occulaires ou dentaires.

Les produits selon l'invention peuvent également trouver une application dans les traitements des troubles cardiovasculaires tels de l'hypotension, ou dans le traitement des troubles liés à une mauvaise régulation de la croissance (nanisme, hypothrophies secondaires à des maladies chroniques de l'enfant, ostéoporose, développement de greffes).

Les doses dépendent de l'effet recherché et de la durée du traitement. Pour un adulte, elles sont généralement comprises entre 0,25 et 1500 mg par jour en prises échelonnées.

D'une façon générale, le médecin déterminera la posologie qu'il estime la plus appropriée en fonction de l'âge, du poids et de tous les autres facteurs propres au sujet à traiter.

Les exemples suivants, donnés à titre non limitatif, illustrent des compositions selon l'invention.

### EXEMPLE A

On prépare, selon la technique habituelle, des comprimés de produit actif ayant la composition suivante :
- [(pyrrolidinyl-1)-3 propoxy-2] phénylacétyl-2 diphényl-4,4 fluoro-7 perhydroisoindole-4-(3aR,7R,7aR) 25 mg
- amidon 83 mg
- silice 30 mg
- stéarate de magnésium 3 mg

### EXEMPLE B

On prépare, selon la technique habituelle, des comprimés de produit actif ayant la composition suivante :
- diphényl-4,4 fluoro-7 [(méthoxy-2 phényl)-2 propionyl-(S)]-2 perhydroisoindole-(3aR,7R,7aR) 25 mg
- amidon 83 mg
- silice 30 mg
- stéarate de magnésium 3 mg

## Revendications

1. Dérivé de perhydroisoindole caractérisé en ce qu'il répond à la formule générale : 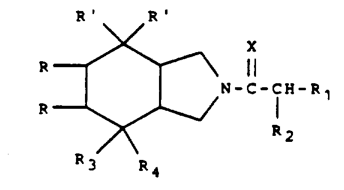 dans laquelle
- les radicaux R sont identiques et représentent des atomes d'hydrogène ou forment ensemble une liaison,
- les symboles R' sont identiques et représentent des radicaux phényle éventuellement substitués par un atome d'halogène ou par un radical méthyle en position 2 ou 3,
- le symbole X représente un atome d'oxygène, ou un radical NH,
- le symbole R₁ représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino) alcoyloxy ou alcoylthio pouvant être éventuellement substitués [par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy, hydroxyalcoyle)], ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle ou hétérocyclyle mono ou polycyclique, saturé ou insaturé contenant 5 à 9 atomes de carbone et un ou plusieurs hétéroatomes choisis parmi l'oxygène, l'azote ou le soufre, et
- le symbole R₂ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino, acylamino ou alcoyloxycarbonylamino,
- le symbole R₃ représente un atome d'halogène ou un radical hydroxy et
- le symbole R₄ représente un atome d'hydrogène ou simultanément à R₃, représente un atome d'halogène,
les radicaux alcoyle et acyle cités ci-dessus étant droits ou ramifiés et contenant 1 à 4 atomes de carbone, sous ses formes stéreoisomères ou leurs mélanges ainsi que ses sels lorsqu'ils existent.

2. Un dérivé de perhydroisoindole selon la revendication 1, caractérisé en ce que
- les radicaux R sont des atomes d'hydrogène,
- les symboles R' sont des radicaux phényle,
- le symbole X représente un atome d'oxygène, ou un radical NH,
- le symbole R₁ représente un radical phényle éventuellement substitué par un radical alcoyloxy pouvant être éventuellement substitué [par un radical dialcoylamino ou dialcoylamino dont les parties alcoyle forment avec l'atome d'azote auquel elles sont rattachées, un hétérocycle à 5 à 6 chaînons], ou substitué par un radical dialcoylamino dont les parties alcoyle peuvent former avec l'atome d'azote auquel elles sont rattachées, un hétérocycle tel que défini dans la revendication 1,
- le symbole R₂ représente un atome d'hydrogène ou un radical alcoyle,
- le symbole R₃ représente un atome de fluor ou de chlore ou un radical hydroxy et
- le symbole R₄ représente un atome d'hydrogène ou simultanément à R₃ représente un atome de fluor,
les radicaux alcoyle cités ci-dessus étant droits ou ramifiés et contenant 1 à 4 atomes de carbone, sous ses formes stéreoisomères ou leurs mélanges ainsi que ses sels lorsqu'ils existent.

3. Le {{[(pyrrolidinyl-1)-3 propoxy-2]phényl}acétyl}-2 diphényl-4,4 fluoro-7 perhydroisoindole, sous ses formes stéreoisomères ou leurs mélanges ainsi que ses sels selon la revendication 1.

4. Le diphényl-4,4 fluoro-7 [(méthoxy-2 phényl)-2 propionyl-(S)]-2 perhydroisoindole sous ses formes stéreoisomères ou leurs mélanges selon la revendication 1.

5. Le [(diméthylamino-3 propoxy)-2 phényl]acétyl-2 diphényl-4,4 fluoro-7 perhydroisoindole sous ses formes stéreoisomères ou leurs mélanges ainsi que ses sels selon la revendication 1.

6. Le diphényl-7,7 [(méthoxy-2 phényl)-2 propionyl-(S)]-2 perhydroisoindolol-4 sous ses formes stéreoisomères ou leurs mélanges selon la revendication 1.

7. Le [(méthoxy-2 phényl)acétyl]-2 diphényl-4,4 fluoro-7 perhydroisoindole sous ses formes stéreoisomères ou leurs mélanges selon la revendication 1.

8. Procédé de préparation d'un nouveau dérivé de perhydroisoindole selon la revendication 1, caractérisé en ce que l'on fait agir un acide ou un dérivé réactif de l'acide de formule générale 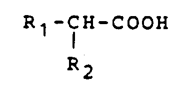 dans laquelle R₁ et R₂ sont définis comme dans la revendication 1, sur un dérivé de l'isoindole de formule générale : 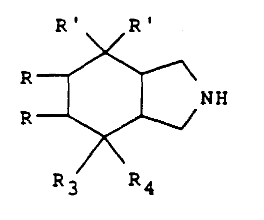 dans laquelle les symboles R, R', R₃ et R₄ sont définis comme dans la revendication 1, puis transforme éventuellement l'amide obtenu en une amidine pour laquelle X représente un radical NH, et transforme éventuellement le produit obtenu en un sel lorsqu'ils existent.

9. Procédé de préparation d'un dérivé perhydroisoindole selon la revendication 1, pour lequel R₃ est un atome d'halogène et R₄ est un atome d'hydrogène, caractérisé en ce que l'on fait agir un agent d'halogénation sur le dérivé correspondant selon la revendication 1, pour lequel R₃ est un radical hydroxy, R₄ est un atome d'hydrogène et X est un atome d'oxygène, puis le cas échéant transforme l'amide obtenu en une amidine et/ou en un sel lorsqu'ils existent.

10. Procédé de préparation d'un dérivé de perhydroisoindole selon la revendication 1 pour lequel R₃ est un radical hydroxy et R₄ est un atome d'hydrogène, caractérisé en ce que l'on réduit un dérivé de perhydroisoindolone de formule générale : 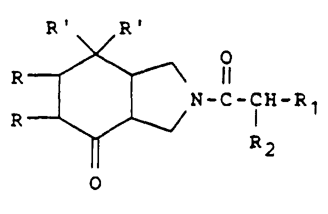 dans laquelle R, R', R₁ et R₂ sont définis comme dans la revendication 1, puis sépare éventuellement les isomères axial et equatorial et le cas échéant transforme l'amide obtenu en une amidine, et/ou en un sel lorsqu'ils existent.

11. Procédé de préparation d'un dérivé de l'isoindolone selon la revendication 1 pour lequel X est un radical NH et les symboles R, R', R₁ , R₂ et R₃ sont définis comme dans la revendication 1, caractérisé en ce que l'on fait agir un produit de formule générale : 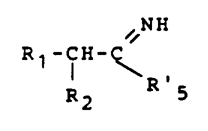 éventuellement à l'état de sel, dans laquelle R₁ R₂ sont définis comme dans la revendication 1 et R'₅ représente un radical alcoyloxy droit ou ramifié contenant 1 à 4 atomes de carbone ou un radical méthylthio, éthylthio, benzylthio ou alcoyloxycarbonylméthylthio, sur un dérivé de l'isoindolone de formule générale : 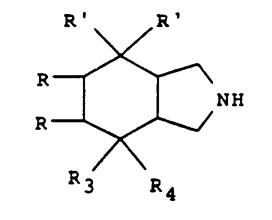 dans laquelle R, R' R₃ et R₄ définis comme dans la revendication 1, puis, lorsqu'ils existent, transforme éventuellement le produit obtenu en un sel.

12. Composition pharmaceutique caractérisée en ce qu'elle contient au moins un produit selon la revendication 1, à l'état pur ou sous forme d'association avec un ou plusieurs adjuvants ou diluants compatibles et pharmaceutiquement acceptables.

## Claims

1. Perhydroisoindole derivative characterised in that it corresponds to the general formula: 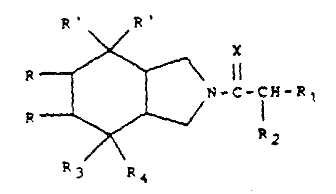 in which
- the R radicals are identical and represent hydrogen atoms or together form a bond,
- the symbols R' are identical and they represent phenyl radicals which are optionally substituted in position 2 or 3 by a halogen atom or by a methyl radical,
- the symbol X represents an oxygen atom or an NH radical,
- the symbol R₁ represents a phenyl radical which is optionally substituted by one or more halogen atoms or hydroxyl or alkyl radicals which may be optionally substituted (by halogen atoms or amino, alkylamino or dialkylamino radicals) alkoxy or alkylthio radicals which may be optionally substituted [by hydroxyl, amino, alkylamino or dialkylamino radicals optionally substituted (by phenyl, hydroxyl or amino radicals) or by dialkylamino radicals whose alkyl parts form with the nitrogen atom to which they are attached, a heterocycle with 5 to 6 members which may contain another heteroatom chosen from oxygen, sulphur or nitrogen, optionally substituted by an alkyl, hydroxyl or hydroxyalkyl radical)], or which is substituted by amino, alkylamino or dialkylamino radicals whose alkyl parts may form with the nitrogen atom to which they are attached, a heterocycle as defined above, or represents a cyclohexadienyl, naphthyl or a saturated or unsaturated, mono- or polycyclic heterocyclic radical containing 5 to 9 carbon atoms and one or more heteroatoms chosen from oxygen, nitrogen or sulphur,
- the symbol R₂ represents a hydrogen or halogen atom or a hydroxyl, alkyl, aminoalkyl, alkylaminoalkyl, dialkylaminoalkyl, alkoxy, alkylthio, acyloxy, carboxyl, alkoxycarbonyl, dialkylaminoalkoxycarbonyl, benzyloxycarbonyl, amino, acylamino or alkoxycarbonylamino radical,
- the symbol R₃ represents a halogen atom or a hydroxyl radical, and
- the symbol R₄ represents a hydrogen atom or, together with R₃, represents a halogen atom,
the alkyl and acyl radicals mentioned above being linear or branched and containing 1 to 4 carbon atoms, in its stereoisomeric forms or mixture thereof, as well as its salts when these exist.

2. Perhydroisoindole derivative according to Claim 1, characterised in that
- the radicals R are hydrogen atoms,
- the symbols R' are phenyl radicals,
- the symbol X represents an oxygen atom or an NH radical,
- the symbol R₁ represents a phenyl radical which is optionally substituted by an alkoxy radical which may be optionally substituted [by a dialkylamino radical or a dialkylamino radical whose alkyl parts form with the nitrogen atom to which they are attached, a heterocycle with 5 to 6 members], or substituted by a dialkylamino radical whose alkyl parts may form with the nitrogen atom to which they are attached, a heterocycle as defined in Claim 1,
- the symbol R₂ represents a hydrogen atom or an alkyl radical,
- the symbol R₃ represents a fluorine or chlorine atom or a hydroxyl radical, and
- the symbol R₄ represents a hydrogen atom or, together with R₃, represents a fluorine atom,
the abovementioned alkyl radicals being linear or branched and containing 1 to 4 carbon atoms, in its stereoisomeric forms or mixtures thereof, as well as its salts when these exist.

3. 2-{{[3-(1-Pyrrolidinyl)-2-propoxy]phenyl}acetyl}-4,4-diphenyl-7-fluoroperhydroisoindole in its stereoisomeric forms or mixtures thereof, as well as its salts according to Claim 1.

4. 4,4-Diphenyl-7-fluoro-2-[(S)-2-(2-methoxyphenyl)propionyl]perhydroisoindole in its stereoisomeric forms or mixtures thereof according to Claim 1.

5. 2-{[2-(3-Dimethylaminopropoxy)phenyl]acetyl}-4,4-diphenyl-7-fluoroperhydroisoindole in its stereoisomeric forms or mixtures thereof, as well as its salts according to Claim 1 according to Claim 1.

6. 7,7-Diphenyl-2-[(S)-2-(2-methoxyphenyl)propionyl]-4-perhydroisoindolol in its stereoisomeric forms or mixtures thereof according to Claim 1.

7. 2-[(2-Methoxyphenyl)acetyl]-4,4-diphenyl-7-fluoroperhydroisoindole in its stereoisomeric forms or mixtures thereof according to Claim 1.

8. Method of preparing a new perhydroisoindole derivative according to Claim 1, characterised in that an acid or a reactive derivative of the acid of general formula 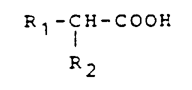 in which R₁ and R₂ are as defined in Claim 1, is reacted with an isoindole derivative of general formula: 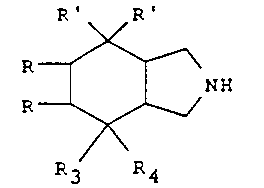 in which the symbols R, R', R₃ and R₄ are as defined in Claim 1, and then the amide obtained is optionally converted to an amidine for which X represents an NH radical and the product obtained is optionally converted to a salt, when these exist.

9. Method of preparing a perhydroisoindole derivative according to Claim 1, for which R₃ is a halogen atom and R₄ is a hydrogen atom, characterised in that a halogenating agent is reacted with the corresponding derivative according to Claim 1, for which R₃ is a hydroxyl radical, R₄ is a hydrogen atom and X is an oxygen atom, and then where appropriate, the amide obtained is converted to an amidine and/or to a salt, when these exist.

10. Method of preparing a perhydroisoindole derivative according to Claim 1, for which R₃ is a hydroxyl radical and R₄ is a hydrogen atom, characterised in that a perhydroisoindolone derivative of general formula: 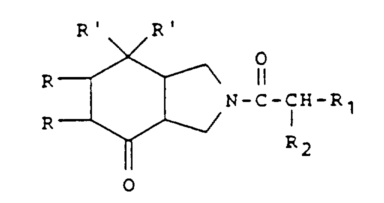 in which R, R', R₁ and R₂ are as defined in Claim 1, is reduced and then the axial and equatorial isomers are optionally separated and, where appropriate, the amide obtained is converted to an amidine and/or to a salt, when these exist.

11. Method of preparing an isoindolone derivative according to Claim 1, for which X is an NH radical and the symbols R, R', R₁, R₂ and R₃ are as defined in Claim 1, characterised in that a product of general formula: 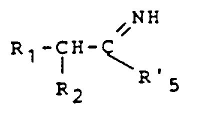 optionally in the form of a salt, in which R₁ and R₂ are as defined in Claim 1, and R'₅ represents a linear or branched alkoxy radical containing 1 to 4 carbon atoms or a methylthio, ethylthio, benzylthio or alkoxycarbonylmethylthio radical, is reacted with an isoindolone derivative of general formula: 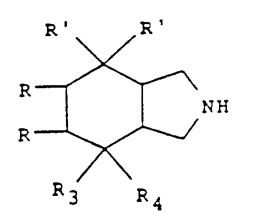 in which R, R', R₃ and R₄ are as defined in claim 1, and then the product obtained is optionally converted to a salt, when these exist.

12. Pharmaceutical composition characterised in that it contains at least one product according to Claim 1, in a pure state or combined with one or more compatible and pharmaceutically acceptable adjuvants or diluents.

## Patentansprüche

1. Perhydroisoindol-Derivat, dadurch gekennzeichnet, daß es der allgemeinen Formel 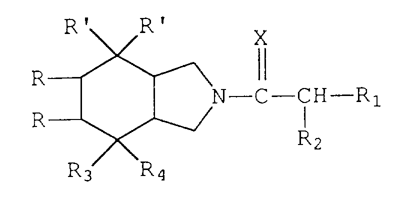 entspricht, in der
- die Reste R identisch sind und Wasserstoffatome darstellen oder zusammen eine Bindung bilden,
- die Symbole R' identisch sind und Phenylreste darstellen, gegebenenfalls substituiert in Position 2 oder 3 durch ein Halogenatom oder durch einen Methylrest,
- das Symbol X ein Sauerstoffatom oder einen Rest NH bedeutet,
- das Symbol R₁ einen Phenylrest darstellt, gegebenenfalls substituiert durch ein oder mehrere Halogenatome oder Hydroxyreste, Alkylreste, die gegebenenfalls substituiert sein können (durch Halogenatome oder die Reste Amino, Alkylamino oder Dialkylamino), Alkyloxy- oder Alkylthioreste, die gegebenenfalls substituiert sein können [durch die Reste Hydroxy, Amino, Alkylamino oder Dialkylamino, gegebenenfalls substituiert (durch die Reste Phenyl, Hydroxy oder Amino), oder Dialkylamino, dessen Alkylteile mit dem Stickstoffatom, an das sie gebunden sind, einen Heterocyclus mit 5 bis 6 Ringgliedern bilden können, der ein anderes Heteroatom enthalten kann, ausgewählt unter Sauerstoff, Schwefel oder Stickstoff, gegebenenfalls substituiert durch einen Rest Alkyl, Hydroxy oder Hydroxyalkyl)], oder substituiert durch die Reste Amino, Alkylamino, Dialkylamino, dessen Alkylteile mit dem Stickstoffatom, an das sie gebunden sind, einen wie oben definierten Heterocyclus bilden können, oder einen Rest Cyclohexadienyl, Naphthyl oder einen mono- oder polycyclischen, gesättigten oder ungesättigten Heterocyclyl-Rest darstellen, der 5 bis 9 Kohlenstoffatome und ein oder mehrere Heteroatome enthält, ausgewählt unter Sauerstoff, Stickstoff oder Schwefel,
- das Symbol R₂ ein Wasserstoffatom oder Halogenatom oder einen Rest Hydroxy, Alkyl, Aminoalkyl, Alkylaminoalkyl, Dialkylaminoalkyl, Alkyloxy, Alkylthio, Acyloxy, Carboxy, Alkyloxycarbonyl, Dialkylaminoalkyloxycarbonyl, Benzyloxycarbonyl, Amino, Acylamino oder Alkyloxycarbonylamino darstellt, und
- das Symbol R₃ ein Halogenatom oder einen Hydroxyrest bedeutet und
- das Symbol R₄ ein Wasserstoffatom oder gleichzeitig mit R₃ ein Halogenatom darstellt,
wobei die oben genannten Reste Alkyl und Acyl gerade oder verzweigt sind und 1 bis 4 Kohlenstoffatome enthalten, in seinen stereoisomeren Formen oder ihren Mischungen sowie seinen Salzen, wenn sie existieren.

2. Perhydroisoindol-Derivat nach Anspruch 1, dadurch gekennzeichnet, daß
- die Reste R Wasserstoffatome sind,
- die Symbole R' Phenylreste darstellen,
- das Symbol X ein Sauerstoffatom oder einen Rest NH bedeutet,
- das Symbol R₁ einen Phenylrest darstellt, gegebenenfalls substituiert durch einen Alkyloxyrest, der gegebenenfalls substituiert sein kann (durch einen Dialkylaminorest oder einen Dialkylaminorest, dessen Alkylteile mit dem Stickstoffatom, an das sie gebunden sind, einen Heterocyclus mit 5 bis 6 Ringgliedern bilden können), oder substituiert durch einen Dialkylaminorest, dessen Alkylteile mit dem Stickstoffatom, an das sie gebunden sind, einen wie in Anspruch 1 definierten Heterocyclus bilden können,
- das Symbol R₂ ein Wasserstoffatom oder einen Alkylrest darstellt,
- das Symbol R₃ ein Fluor- oder Chloratom oder einen Hydroxyrest bedeutet und
- das Symbol R₄ ein Wasserstoffatom oder gleichzeitig mit R₃ ein Fluoratom darstellt,
wobei die oben genannten Alkylreste gerade oder verzweigt sind und 1 bis 4 Kohlenstoffatome enthalten,
in seinen stereoisomeren Formen oder ihren Mischungen sowie seinen Salzen, wenn sie existieren.

3. 2-{{[3-(1-Pyrrolidinyl)-2-propoxy]-phenyl}-acetyl}-4,4-diphenyl-7-fluor-perhydroisoindol in seinen stereoisomeren Formen und ihren Mischungen sowie seinen Salzen nach Anspruch 1.

4. 2-[2-(2-Methoxyphenyl)-propionyl-(S)]-4,4-diphenyl-7-fluorperhydroisoindol in seinen stereoisomeren Formen und ihren Mischungen nach Anspruch 1.

5. 2-[2-(3-Dimethylamino-propoxy)-phenyl]-acetyl-4,4-diphenyl-7-fluor-perhydroisoindol in seinen stereoisomeren Formen und ihren Mischungen sowie seinen Salzen nach Anspruch 1.

6. 2-[2-(2-Methoxyphenyl)-propionyl-(S)]-7,7-diphenyl-4-perhydroisoindol in seinen stereoisomeren Formen und ihren Mischungen nach Anspruch 1.

7. 2-[(2-Methoxyphenyl)-acetyl]-4,4-diphenyl-7-fluor-perhydroisoindol in seinen stereoisomeren Formen und ihren Mischungen nach Anspruch 1.

8. Verfahren zur Herstellung eines neuen Perhydroisoindol-Derivates nach Anspruch 1, dadurch gekennzeichnet, daß man eine Säure oder ein reaktives Derivat der Säure der allgemeinen Formel 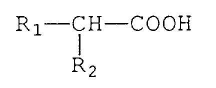 in der R₁ und R₂ wie in Anspruch 1 definiert sind, mit einem Isoindol-Derivat der allgemeinen Formel 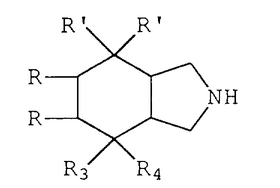 zur Reaktion bringt, in der die Symbole R, R', R₃ und R₄ wie in Anspruch 1 definiert sind, anschließend das erhaltene Amid gegebenenfalls in ein Amidin umwandelt, in dem X einen Rest NH darstellt, und gegebenenfalls das erhaltene Produkt in ein Salz umwandelt, wenn es existiert.

9. Verfahren zur Herstellung eines Perhydroisoindol-Derivates nach Anspruch 1, worin R₃ ein Halogenatom ist und R₄ ein Wasserstoffatom darstellt, dadurch gekennzeichnet, daß man ein Halogenierungsmittel mit dem entsprechenden Derivat von Anspruch 1 zur Reaktion bringt, worin R₃ ein Hydroxyrest ist, R₄ ein Wasserstoffatom darstellt und X ein Sauerstoffatom bedeutet, anschließend das erhaltene Amid gegebenenfalls in ein Amidin und/oder in ein Salz umwandelt, wenn es existiert.

10. Verfahren zur Herstellung eines Perhydroisoindol-Derivates nach Anspruch 1, worin R₃ ein Hydroxyrest ist und R₄ ein Wasserstoffatom darstellt, dadurch gekennzeichnet, daß man ein Perhydroisoindol-Derivat der allgemeinen Formel 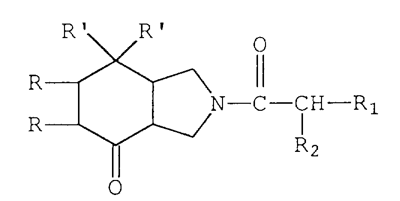 in der R, R', R₁ und R₂ wie in Anspruch 1 definiert sind, reduziert, anschließend gegebenenfalls die Isomeren axial und äquatorial trennt und gegebenenfalls das erhaltene Amid in ein Amidin und/oder in ein Salz umwandelt, wenn es existiert.

11. Verfahren zur Herstellung eines Isoindolon-Derivates nach Anspruch 1, worin X einen Rest NH darstellt und die Symbole R, R', R₁, R₂ und R₃ wie in Anspruch 1 definiert sind, dadurch gekennzeichnet, daß man ein Produkt der allgemeinen Formel 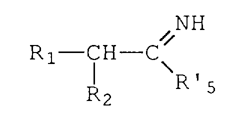 gegebenenfalls im Zustand des Salzes, worin R₁ und R₂ wie in Anspruch 1 definiert sind und R^{'}₅ einen geraden oder verzweigten Alkyloxyrest mit 1 bis 4 Kohlenstoffatomen oder einen Rest Methylthio, Ethylthio, Benzylthio oder Alkyloxycarbonylmethylthio darstellt, mit einem Isoindol-Derivat der allgemeinen Formel 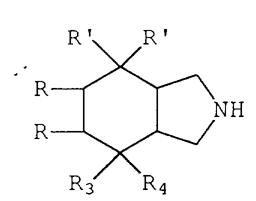 zur Reaktion bringt, in der die Symbole R, R', R₃ und R₄ wie in Anspruch 1 definiert sind und anschließend das Produkt gegebenenfalls in ein Salz umwandelt, wenn es existiert.

12. Pharmazeutische Zusammensetzungen, dadurch gekennzeichnet, daß sie mindestens ein Produkt nach Anspruch 1 im reinen Zustand oder in Form einer Assoziation mit einem oder mehreren kompatiblen und pharmazeutisch akzeptablen Zusatzstoffen oder Verdünnungsmitteln enthalten.
